# FASCICULE DE BREVET EUROPEEN

(11) **EP 3 021 669 B1**
(45) Date de publication et mention de la délivrance du brevet: **28.04.2021**
(21) Numéro de dépôt: 14749932.1
(22) Date de dépôt: 21.07.2014
(51) Int. Cl.: A01N 31/06, C07H 15/26, A61K 31/4375, A61K 31/70, C07D 487/04

(54) **NOUVEAUX COMPOSES DÉRIVÉS DE LA SOLANIDINE**
NEUARTIGE , VON SOLANIDIN ABGELEITETE VERBINDUNGEN
NOVEL SOLANIDINE-DERIVED COMPOUNDS

(30) Priorité: 19.07.2013 FR 1357153
(43) Date de publication de la demande: 25.05.2016
(73) Titulaire: Université de Picardie Jules Verne, 80025 Amiens Cedex 1 (FR); Semences Innovation Protection Recherche et Environnement, 62217 Achicourt (FR)
(72) Inventeur: BEAULIEU, Rémi, F-62217 Achicourt (FR); ATTOUMBRE, Jacques, F-62217 Achicourt (FR); GOBERT-DEVEAUX, Virginie, F-62217 Achicourt (FR); GRAND, Eric, F-80025 Amiens Cedex 1 (FR); STASIK, Imane, F-80025 Amiens Cedex 1 (FR); KOVENSKY, José, F-80025 Amiens Cedex 1 (FR); GIORDANENGO, Philippe, F-80025 Amiens Cedex 1 (FR)
(74) Mandataire: Fédit-Loriot
(86) Numéro de dépôt international: PCT/FR2014/051871
(87) Numéro de publication internationale: WO 2015/008007

(56) Documents cités:
- WO-A1-98/58543
- WO-A2-01/49279
- WO-A2-2011/068987
- GOMAH E. NENAAH: "Toxic and antifeedant activities of potato glycoalkaloids against Trogoderma granarium (Coleoptera: Dermestidae)", JOURNAL OF STORED PRODUCTS RESEARCH, vol. 47, no. 3, 1 juillet 2011 (2011-07-01), pages 185-190, XP055088298, ISSN: 0022-474X, DOI: 10.1016/j.jspr.2010.11.003

## Description

La présente invention concerne de nouveaux composés dérivés de glycoalcaloïdes, leur procédé de synthèse, et leurs utilisations notamment dans le domaine de la protection phytosanitaire et de la santé. En particulier, la présente invention concerne de nouveaux composés présentant des propriétés toxiques et/ou répulsives sur les pucerons ainsi que d'autres propriétés.

Les glycoalcaloïdes sont des molécules naturelles métabolisées exclusivement par les plantes. Bien que certaines de ces molécules puissent être isolées des plantes de la famille des Liliaceae, on les trouve essentiellement dans la famille des Solanaceae comprenant des espèces alimentaires telles que la pomme de terre, l'aubergine et la tomate.

Le développement de l'industrie de la pomme de terre s'accompagne d'une augmentation inévitable de la quantité de coproduits. La gestion de ces déchets représente un enjeu à la fois environnemental, économique et réglementaire. Aujourd'hui, les coproduits de cette filière sont exploités principalement dans l'alimentation animale. Il est donc essentiel d'ouvrir le potentiel de cette biomasse à d'autres filières et d'accroître leur valeur ajoutée.

La pomme de terre biosynthétise des glycoalcaloïdes tels que la chaconine et la solanine qui se trouvent en quantité non négligeable dans les coproduits industriels. Ce sont des métabolites secondaires qui servent à la protection naturelle de la plante contre les attaques des phytopathogènes. Elles sont notamment efficaces contre les aphides, les coléoptères, la cicadelle, les nématodes, et les champignons.

Les chaconine et solanine possèdent une activité cytotoxique en agissant sur la membrane cellulaire. Deux voies d'action ont été mises en avant : une action de lyse de la membrane elle-même et une action d'inhibition des transports transmembranaires. De plus, elles ont la capacité d'inhiber deux enzymes essentielles à la transmission nerveuse c'est-à-dire, l'acétylcholinestérase (AChe) et la butyrylcholinestérase (BuChe).

On pourra se référer non seulement à l'article intitulé : " Toxic and antifeedant activities of patato glycoalkaloids against Togoderme granarium (Coleoptera: Dermestidae) ", de Gomah E. Nenaah paru dans le Journal of Stored Products Research, Vol. 47, n°3, 1 juillet 20011, mais aussi aux documents WO01/49279 A2, WO98/58543 A1 et WO2011/068987 A2.

Bien qu'elles montrent une efficacité intéressante contre les nuisibles, certaines espèces ont développé des mécanismes de résistance à de tels glycoalcaloïdes.

La présente invention a été réalisée par rapport à l'art antérieur décrit ci-dessus, et le but de la présente invention est, non seulement d'obtenir de nouveaux composés plus efficaces que les glycoalcaloïdes naturels, qui présentent une stabilité et des propriétés biologiques accrues, mais aussi de fournir un nouveau procédé de fabrication desdits composés. De surcroit, la présente invention a également pour but de trouver de nouvelles utilisations de tels composés.

Dans le but de résoudre ce problème, la présente invention propose un composé selon la revendication 1.

Les molécules de monosaccharide sont liées entre elles soit par une liaison O-glycosidique, soit par une liaison S-glycosidique. De préférence, toutes les molécules de monosaccharide sont liées entre elles par une liaison O-glycosidique.

De préférence, ladite unité saccharidique est un hexose.

De préférence, R₁ est un groupe hexosyle. De manière avantageuse, R₁ est choisi parmi le groupe consistant en un glucosyle, un galactosyle et un rhamnosyle.

Ainsi, une caractéristique de l'invention réside dans une mise en œuvre d'un nouveau bras espaceur (linker) entre les différents motifs saccharidiques et la solanidine. Grâce à cette caractéristique, le composé selon l'invention possède une stabilité et des propriétés biologiques accrues. Les présents inventeurs ont découvert que le bras espaceur selon l'invention pourrait avoir plusieurs rôles. Le premier serait d'éloigner suffisamment la partie saccharidique de la partie aglycone responsable de l'activité. Lorsque ces deux parties sont très proches, une gêne stérique pourrait limiter l'activité inhibitrice sur les enzymes. On pourrait donc éviter que sa réactivité soit réduite par encombrement stérique. Le deuxième rôle serait d'apporter de la flexibilité aux composés. En effet, la chaconine et la solanine sont des molécules plutôt rigides et l'apport de flexibilité serait bénéfique dans le cas de l'activité cytotoxique. Grâce à cette flexibilité, un glycoalcaloïde composé d'un monosaccharide, d'un disaccharide ou d'un thio-disaccharide pourrait suffire à provoquer les propriétés membranolytiques (déjà démontrées dans la littérature). De surcroit, le triazole est plus stable en milieu biologique que la plupart des autres liaisons utilisées pour les bras espaceurs.

De préférence, X est un atome d'oxygène, ou un atome de soufre.

Selon la présente invention, on entend par « hexose », des composés de formule chimique C₆H₁₂O₆ cycliques tels que le glucose et le galactose, ou des désoxyhexoses de formule chimique C₆H₁₂O₅ cycliques tels que le rhamnose et le fucose.

Selon la présente invention, on entend par « pentose », des composés de formule chimique C₅H₁₀O₅ cycliques tels que l'arabinose, le ribose ou le xylose, ou des désoxypentoses de formule chimique C₅H₁₆O₄ cycliques tel que le désoxyribose.

Dans le cadre de la présente invention, le « disaccharide » est formé par une combinaison de deux molécules de monosaccharide. Les deux monosaccharides sont liés entre eux par une liaison osidique. A titre d'exemple, le disaccharide peut être le saccharose, le maltose, ou bien le lactose.

Dans le cadre de la présente invention, le « trisaccharide » est formé par une combinaison de trois molécules de monosaccharide, lesquelles sont liées entre elles par une liaison osidique. A titre d'exemple, le trisaccharide peut être le chacotriose ou le solatriose.

Au sens de la présente invention, le « thio-disaccharide » est formé par une combinaison de deux molécules de monosaccharides liées entre elles par une liaison S-glycosidique.

Au sens de la présente invention, le « thio-trisaccharide » est formé par une combinaison de trois molécules de monosaccharides dans laquelle au moins deux molécules de monosaccharides sont liées entre elles par une liaison S-glycosidique.

La présente invention concerne également un composé intermédiaire de formule (II) : 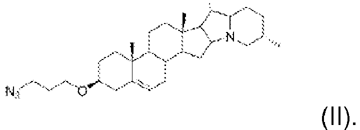 Ainsi, les composés de formule (I) selon l'invention peuvent être obtenus à partir de ce composé.

La présente invention concerne également diverses utilisations du composé de formule (I). De préférence, le composé de formule (I) est utilisé en tant qu'insecticide. Le composé selon l'invention peut notamment être utilisé en tant qu'aphicide.

La présente invention concerne en outre, un procédé d'obtention du composé de formule (I). Selon l'invention le procédé comprend les étapes suivantes :
a) on fournit ledit composé de formule (II) : 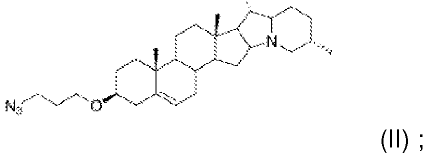 et
b) on fait réagir, en présence d'un catalyseur, ledit composé de formule (II) avec un composé de formule (III) : 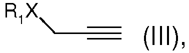 dans laquelle R₁ est tel que défini précédemment.

Ainsi, les présents inventeurs ont mis au point le couplage entre la partie saccharidique (composé III) et la partie aglycone (composé II).

En ce qui concerne l'étape (a), le composé de formule (II) c'est-à-dire la 3-*O*-(3-azidopropyl)solanidine est préparée par un procédé adapté.

S'agissant de l'étape (b), le catalyseur est choisi parmi des composés de cuivre ou de ruthénium. De préférence, le couplage par la cycloaddition alcyne-azoture catalysée au cuivre (CuAAC) est effectué selon l'une des méthodes décrites par Sharpless (Sharpless et al., Angew. Chem. Int. Ed. 2002, 41 (14), 2596-2599) et Meldal (Meldal et al., J. Org. Chem. 2002, 67 (9), 3057-3064). Cette cycloaddition alcyne-azoture possède de nombreux avantages en termes de réactivité. Elle est compatible avec de nombreuses fonctions chimiques, elle est parfaitement régiosélective, elle n'entraîne la formation d'aucun produit secondaire et donne en règle générale des rendements élevés. Lorsque l'on utilise la méthode de couplage par CuAAC de Meldal pour la synthèse (selon Zhang et al., Synthetic Commun. 2009, 39 (5), 830-844), il est indispensable d'utiliser un solvant organique comme le toluène ou le THF. Dans ces solvants, la solubilité des sucres libres (hydroxyles non substitués) est limitée et impose de réaliser le couplage sur des sucres protégés (hydroxyles substitués par des groupements protecteurs). Tandis que lorsque l'on utilise la méthode de couplage par CuAAC de Sharpless *et al.* pour la synthèse de glycostéroïdes (selon Rivera & al. Tetrahedron 2011, 67 (40), 7713-7727), un mélange de solvant organique et d'eau est nécessaire à la réduction du cuivre (II) en cuivre (I). La présence d'eau permet aussi de solubiliser très facilement les sucres libres. Le clivage des groupements protecteurs est donc réalisé avant la CuAAC. La méthode de Sharpless utilise le cuivre (II), en quantité stoechiométrique, qui est réduit *in situ* en cuivre (I) à l'aide d'ascorbate de sodium. La méthode de Meldal utilise directement le cuivre (I) mais en quantité catalytique. De manière avantageuse, le catalyseur est choisi parmi des sels de cuivre (II), de préférence, le catalyseur est CuSO₄.

En outre, il est préférable de purifier les glycoalcaloïdes après l'étape b). La purification des composés selon l'invention peut être effectuée par Chromatographie de Partage Centrifuge (CPC).

De manière encore plus avantageuse, le procédé d'obtention du composé de formule (I) comprend en outre les étapes suivantes :
c) on fournit ledit composé de formule (IV) : 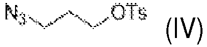 dans laquelle Ts est un groupe tosyle ;
d) on fournit un composé de formule (V) : 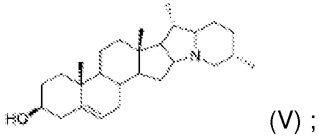 et
e) on fait réagir le composé de formule (IV) avec le composé de formule (V) pour obtenir le composé de formule (II).

Le composé de formule (V), c'est-à-dire la solanidine est obtenu par un procédé adapté connu de l'homme du métier. Il peut, par exemple être obtenu à partir de la chaconine ou de la solanine. On hydrolyse les chaconine et/ou solanine qui sont extraites des coproduits issus de l'industrie de la pomme de terre. Une fois débarrassée de la partie saccharidique par hydrolyse, la solanidine peut être purifiée par CPC.

Étant donné que la solanidine, issue de l'extraction sur les coproduits, n'est disponible qu'en faible quantité, il y avait un réel besoin de fournir un procédé permettant d'obtenir le composé de formule (II) avec un rendement relativement important. Grâce au procédé selon l'invention, le rendement du composé de formule (II), peut atteindre plus de 70 %. Le couplage du composé de la formule (IV) avec le composé de formule (V) peut être effectué dans des conditions définies par l'homme du métier.

La présente invention concerne également une composition comprenant du composé de formule (I). Dans ce cas, la composition comprend au moins 0,001 mM, de préférence, de 0,001 à 2 mM de composé de formule (I).

D'autres particularités et avantages de l'invention ressortiront à la lecture de la description faite ci-après d'un mode de réalisation particulier de l'invention, donné à titre indicatif mais non limitatif, en référence aux dessins annexés sur lesquels :
- la Figure 1A représente des effets des composés selon l'invention (composés 100, 101 et 102) sur la survie larvaire de *Macrosiphum euphorbiae* dans laquelle les résultats ont été analysés par un test χ² de Pearson ; α = 0,05 ; * = différence significative par rapport au témoin ; ‡ = différence significative par rapport à la solanidine à 0,2 mM ;
- la Figure 1B représente des effets de la chaconine, de la solanine et de la solanidine sur la survie larvaire de *Macrosiphum euphorbiae* dans laquelle les résultats ont été analysés par un test χ² de Pearson ; α = 0,05 ; * = différence significative par rapport au témoin ;
- la Figure 2 illustre des effets des composés selon l'invention sur *Macrosiphum euphorbiae* adulte dans laquelle les résultats ont été analysés par un test χ² de Pearson ; α = 0,05 ; * = différence significative par rapport au témoin ;
- la Figure 3 représente des effets des composés selon l'invention sur la reproduction de *Macrosiphum euphorbiae.*

### Exemple 1 Préparation des composés selon l'invention

### 1-1. Matériels et méthodes chimiques

### a) Réactifs et solvants

Les réactifs utilisés proviennent des sociétés Sigma-Aldrich, Acros et Alfa Aesar et sont utilisés sans purification. Les solvants anhydres, à l'exception du dichlorométhane et du THF, sont achetés chez Acros sous le format Acroseal®. Le dichlorométhane est distillé sous atmosphère inerte en présence d'hydrure de calcium. Le THF est distillé sous atmosphère inerte en présence de sodium et de benzophénone.

### b) Chromatographies

### Chromatoaraphie sur Couche Mince (CCM)

Les chromatographies sur couche mince sont effectuées sur des plaques de silice avec support d'aluminium Merck 60 F₋₂₅₄. Les techniques utilisées pour révéler les produits après élution sont :
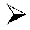 pour les dérivés saccharidiques : H₂O / H₂SO₄ à 96 % / (NH₄)₄Ce(SO₄), 2 H₂O/ (NH₄)₆Mo₇O₂₄, 4H₂O (188 mL / 12 mL / 2,1 g / 5,3 g) puis chauffage.
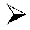 pour les dérivés de solanidine : Dragendorf selon Munier et Macheboeuf.

Solution A : dissoudre 0,85 g de nitrate de bismuth dans 10 mL d'acide acétique et 40 mL d'eau.
Solution B : dissoudre 8 g d'iodure de potassium dans 20 mL d'eau.
Solution de vaporisation : solution A / solution B / AcOH / H₂O (1 mL / 1 mL / 4 mL / 20 mL).

### Chromatographie flash manuelle

Les purifications manuelles sont réalisées à l'aide de colonnes en verre remplies de gel de silice (GEDURAN SI 60, granulométrie 0,040-0,062 mm Merck). Le brut réactionnel est dissous dans un minimum de solvant pour être déposé en tête de colonne ou adsorbé sur de la silice avant le dépôt en tête de colonne. L'élution est réalisée par le passage d'un gradient d'éluants sous pression d'air (0,8 bar).

### Chromatographie flash automatisée

Les purifications flash automatiques ont été réalisées avec un appareil à chromatographie flash Grace (Reveleris® iES Flash Sytem). La séparation se fait à l'aide de colonnes, de silice ou C-18, prépaquées disponibles dans le commerce (4g, 12g, 24g, 40g, 80g). Il est possible de réaliser des purifications en phase normale, mais aussi en phase inverse. La masse de produits purifiables correspond au maximum à 10 % de la masse de silice de la colonne prépaquée. Les purifications sont réalisées à une pression moyenne de 50 psi (pouvant monter jusqu'à 200 psi). Cet appareil est équipé d'un détecteur à diffusion de lumière (DDL) et d'un détecteur UV à deux longueurs d'onde paramétrables.

### Chromatogaphie par CPC

Les purifications par Chromatographie de Partage Centrifuge ont été réalisées avec un appareil Armen® CPC 250 relié à un collecteur Armen® LS-5600. C'est un appareil semi-préparatif dans lequel il est possible d'injecter jusqu'à 6 g de produit brut. Les produits sont séparés en fonction de leur coefficient de partage dans un mélange de solvants biphasique donné. Lorsque la phase lourde est maintenue stationnaire alors que la phase mobile est injectée en flux continu on parle de mode ascendant. Dans le cas inverse, on parle de mode descendant. L'appareil est équipé d'un détecteur UV-visible.

En ce qui concerne la solanidine, la purification est réalisée en mode ascendant dans un mélange biphasique de H₂O / BuOH / AcOEt : 50 / 7,5 / 42,5. La centrifugation est réalisée à 3000 rpm.

### c) Caractérisation des composés

### Le pouvoir rotatoire spécifique

Les pouvoirs rotatoires spécifiques sont mesurés à une température de 20 °C à l'aide du polarimètre Perkin-Elmer 343 qui émet une lumière polarisée de longueur d'onde λ=549 nm (raie D du sodium). La concentration c est exprimée en grammes pour 100 mL de solvant.

### Point de fusion

La mesure des points de fusion est effectuée à l'aide d'un appareil automatique Büchi 535 avec une température maximale de 275 °C.

### Spectrométrie infrarouge

Les analyses par spectrométrie infrarouge sont réalisées sur un spectromètre Shimadzu® FTIR-84005. Les mesures sont effectuées par la technique ATR avec le logiciel IR solution et les valeurs des bandes obtenues sont exprimées en cm-1.

### Spectrométrie de masse basse résolution

Les analyses en spectrométrie de masse basse résolution sont réalisées sur un appareil simple quadrupôle (Micromass-Waters ZQ) possédant une source d'ionisation électrospray (Z-spray). L'utilisation de cet appareil permet l'utilisation du mode d'ionisation positif ou négatif. Le voltage de capillaire est de 3,5 kV et la tension de cône a été variée entre |20| et |120| V. Les températures de la source et de désolvatation sont respectivement de 80 et 150 °C. Le gaz de désolvatation et de nébulisation est l'azote.

### Spectrométrie de masse haute résolution

Les analyses en spectrométrie de masse haute résolution (HRMS) sont réalisées sur un spectromètre de masse hybride quadrupôle à temps de vol (Micromass-Waters Q-TOF Ultima Global), équipé d'une source d'ionisation électrospray. Les températures de la source et de désolvatation sont respectivement de 80 et 120 °C. Le gaz utilisé comme gaz de nébulisation et de désolvatation est l'azote avec un débit respectif de 20 et 500 L/h. Le voltage du capillaire est de 3,5 kV et la tension de cône a été variée entre 100 et 250 V. Avant toute mesure de masse exacte, une calibration est effectuée avec de l'acide orthophosphorique. La précision de la mesure de masse exacte du Q-TOF étant inférieure à 5 ppm, l'accès à la composition élémentaire des molécules est possible.

### Spectrométrie RMN

Les spectres RMN ¹H et RMN ¹³C sont réalisés par deux spectromètres RMN. Le premier est un spectromètre Bruker avance 300. Les spectres RMN ¹H sont enregistrés à 300 MHz et les spectres RMN ¹³C à 75 MHz. La sonde utilisée est une sonde QNP 5 mm. Le deuxième est un spectromètre Bruker avance 600. Dans ce cas, les spectres RMN ¹H sont enregistrés à 600 MHz et les spectres RMN ¹³C à 150 MHz. La sonde utilisée est une sonde TXi 5 mm. Toutes les expériences ont été réalisées à une température autour de 25 °C dans un solvant deutéré qui sert aussi de référence, à l'exception du D₂O où une goutte de méthanol sert de référence pour la RMN ¹³C (voir tableau 1 suivant). Les déplacements chimiques sont donnés en ppm. Les constantes de couplages sont exprimées en Hertz. Les spectres RMN sont traités à l'aide de TopSpin ou MestReNova.

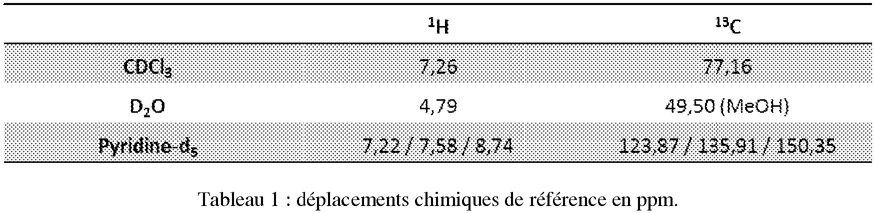

### Numérotation générale des carbones :

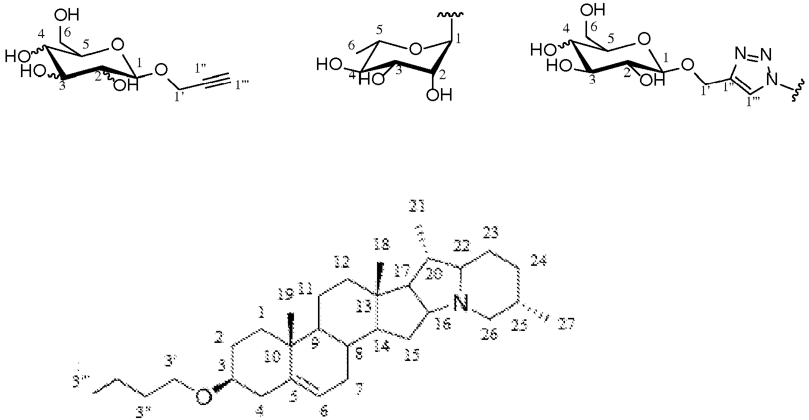

### 1-2. Etapes préparatoires

### Composé 2 : 2,3,4,6-tétra-O-acétyl-β-D-glucopyranoside de propargyle de formule:

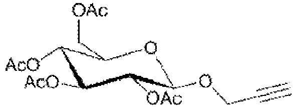

Le 1,2,3,4,6-penta-*O*-acétyl-β-D-glucopyranose (25,62 mmol, 10,0 g) est dissous dans du dichlorométhane anhydre (3,12 mol, 200 mL). Le milieu réactionnel est ensuite mis sous atmosphère inerte. L'alcool propargylique (30,72 mmol, 1,82 mL) est ajouté ainsi que le BF₃.Et₂O (102,48 mmol, 12,64 mL). Le mélange est agité pendant 2 heures toujours sous atmosphère inerte et à température ambiante. On ajoute du carbonate de potassium (38,43 mmol, 5,31 g) et on laisse agiter pendant 30 minutes. Le milieu réactionnel est filtré sur verre fritté et le filtrat jaune récupéré est lavé avec deux fois 200 mL d'eau distillée. Les phases aqueuses sont rassemblées et extraites avec deux fois 100 mL de dichlorométhane. Les phases organiques sont réunies et séchées sur du MgSO₄. Le solvant est évaporé sous pression réduite. Le solide obtenu est dissous dans du dichlorométhane, puis cristallisé par ajout de cyclohexane jusqu'à apparition d'un trouble. On laisse agiter 20 minutes puis on filtre sur verre fritté. La cristallisation est reproduite 2 fois. On obtient un solide blanc que l'on sèche une nuit au dessiccateur. Le composé 2 est ainsi obtenu avec un rendement de 84 % (8,3 g).
**Aspect :** solide blanc
**M :** 386,35 g.mol⁻¹
**Formule brute** : C₁₇H₂₂O₁₀
**Rf :** 0,32 (toluène / AcOEt : 75 / 25)
**Pf :** 114-115 °C
**[α]_{D}²⁰ :** -43,2° (c=1, CHCl₃)
**ESI-MS (M** + **Na+) :** 409
**FT-IR (ATR en cm⁻¹) :** 3273 (_{UC≡H}), 1753-1732 (uc=o), 1232-1207 (_{UC-O}), 1037 (_{UC-O})
**RMN ¹H (CDCl₃, 300 MHz)** δ 5.19 (t, 1H, ³J_{3,2} = ³J_{3,4} = 9.6 Hz, H₃), 5.05 (t, 1H, ³J_{4,3} = ³J_{4,5} = 9.6 Hz, H₄), 4.96 (dd, 1H, ³J_{2,3} = 9.6 Hz, ³J_{2,1} = 7.8 Hz, H₂), 4.73 (d, 1H, ³J_{1,2} = 7.8 Hz, H₁), 4.32 (d, 2H, ⁴J_{1',1'''} = 2.4 Hz, H_{1'}), 4.23 (dd, 1H, ²J_{6a,6b} = 12.3 Hz, ³J_{6a,5} = 4.5 Hz, H₆ₐ), 4.09 (dd, 1H, ²J_{6b,6a} = 12.3 Hz, ³J_{6b,5} = 2.4 Hz, H_{6b}), 3.69 (ddd, 1H, ³J_{5,4} = 9.6 Hz, ³J_{5,6a} = 4.5 Hz, ³J_{5,6b} = 2.4 Hz, H₅), 2.45 (t, 1H,⁴J_{1''',1'} = 2.4 Hz, H_{1'''}), 2.04-1.96 (4s, 12H, CH₃CO₂)
RMN **¹³C (CDCl₃,** 75 MHz) δ 170.64-169.42 (CH₃CO₂), 98.16 (C₁), 78.16 (C_{1"}), 75.59 (C_{1'''}), 72.78 (C₃), 71.94 (C₅), 70.98 (C₂), 68.33 (C₄), 61.79 (C₆), 55.96 (C_{1'}), 20.73-20.61 (CH₃CO₂)

### Composé 3 : β-D-glucopyranoside de propargyle de formule :

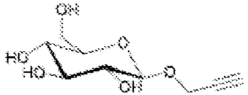

Le composé 2 (23,0 mmol, 8,9 g) est dissous dans du méthanol (4,9 mol, 200 mL). Une solution de sodium (20,0 mmol, 465 mg) dans du méthanol (4,9 mol, 200 mL) est ensuite ajoutée au milieu réactionnel. Le mélange est agité pendant 15 h à température ambiante. On ajoute ensuite une résine échangeuse d'ion IR-120 [H+] jusqu'à pH =5-6. Le milieu réactionnel est filtré sur verre fritté et la résine est lavée au méthanol. Le solvant est évaporé sous pression réduite et le solide séché une nuit au dessiccateur. Le composé 3 est obtenu avec un rendement de 98 % (4,9 g).
**Aspect :** solide blanc
**M :** 218,20 g.mol⁻¹
**Formule brute :** C₉H₁₄O₆
**Rf :** 0,43 (CH₂Cl₂ / MeOH : 80 / 20)
**Pf :** 54-55 °C
**[α]_{D}²⁰ :** -53,9° (c=2, H2O)
**ESI-MS (M + Na+) :** 241
**FT-IR (ATR en cm⁻¹) :** 3289 (_{UO-H}), 3236 2874 (_{UC-H}), 1365 (_{UC-O}), 1026 (_{UC-O})
**RMN ¹H (D₂O, 300 MHz)** δ 4.61 (d, 1H, ³J_{1,2} = 8.1 Hz, H₁), 4.54 (dd, 1H, ²J_{1'a,1'b} = 15.9 Hz, ⁴J_{1'a,1'''} = 2.4 Hz, H_{1'a}), 4.48 (dd, 1H, ²J_{1'b,1'a} = 15.9 Hz, ⁴J_{1'b,1'''} = 2.4 Hz, H_{1'b}), 3.95 (dd, 1H, ²J_{6a,6b} = 12.3 Hz, ³J_{6a,5} = 2.2 Hz, H₆ₐ), 3.76 (dd, 1H, ²J_{6b,6a} = 12.3 Hz, ³J_{6b,5} = 5.7 Hz, H_{6b}), 3.50 (m, 3H, H₃, H₄ et H₅), 3.33 (dd, 1H, ³J_{2,1} = 8.1 Hz, ³J_{2,3} = 9.0 Hz, H₂), 2.99 (t, 1H, ⁴J_{1''',1'} = 2.4 Hz, H_{1'''})
**RMN ¹³C (D₂O, 75 MHz)** δ 101.09 (C₁), 79.45 (C_{1''}), 76.98 (C_{1'''}), 76.51 (C₃, C₄ ou C₅), 76.25 (C₃, C₄ ou C₅), 73.42 (C₂), 70.10 (C₃, C₄ ou C₅), 61.26 (C₆), 57.08 (C_{1'})

### Composé 5: 2,3,4,6-tétra-O-acétyl-β-D-galactopyranoside de propargyle de formule :

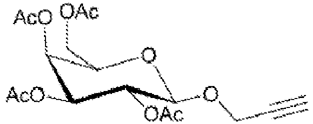

Le 1,2,3,4,6-penta-O-acétyl-β-D-galactopyranose (12,81 mmol, 5,0 g) et du trifluoroacétate d'argent (19,21 mmol, 4,28 g) sont placés sous atmosphère inerte. On ajoute du dichlorométhane anhydre (2, 34 mol, 150 mL), puis l'alcool propargylique (19,21 mmol, 1,13 mL) et le chlorure d'étain (38,43 mmol, 4,5 mL). La solution est agitée pendant 1 h 30 sous atmosphère inerte à température ambiante. Le milieu réactionnel est dilué par ajout de 400 mL de dichlorométhane puis lavé avec, successivement, 300 mL d'une solution saturée de NaHCO3, trois fois 300 mL d'eau distillée et enfin 300 mL d'une solution saturée de NaCl. La phase organique est séchée sur du MgSO4 et les solvants sont évaporés sous pression réduite. Le sirop jaune obtenu est purifié par chromatographie manuelle sur gel de silice avec un gradient d'éluant AcOEt / cyclohexane : 20 / 80 à 40 / 60. Le résidu est séché une nuit au dessiccateur. Le composé 5 est ainsi obtenu avec un rendement de 87 % (4,3 g).
**Aspect :** Solide blanc
**M :** 386,35 g.mol⁻¹
**Formule brute :** C₁₇H₂₂O₁₀
**Rf :** 0,47 (cyclohexane / AcOEt : 50 / 50)
**Pf :** 66-68 °C
**[α]_{D}²⁰ :** -33,4° (c=1, CHCl₃)
**ESI-MS (M** + **Na+) :** 409
**FT-IR (ATR en cm⁻¹) :** 3276 1741 (uc=o), 1212-1044 (_{UC-O})
**RMN ¹H (CDCl₃, 300 MHz)** δ 5.30 (dd, 1H, ³J_{4,3} = 3,4 Hz, ³J_{4,5} = 1.0 Hz, H₄), 5.11 (dd, 1H, ³J_{2,3} = 10.5 Hz, ³J_{2,1} = 7.9 Hz, H₂), 4.96 (t, 1H, ³J_{3,2} = 10.5 Hz, ³J_{3,4} = 3.4 Hz, H₃), 4.65 (d, 1H, ³J_{1,2} = 7.9 Hz, H₁), 4.28 (d, 2H, ⁴J_{1',1'''} = 2.4 Hz, H_{1'}), 4.09 (dd, 1H, ²J_{6a,6b} = 11.2 Hz, ³J_{6a,5} = 6.6 Hz, H₆ₐ), 4.04 (dd, 1H, ²J_{6b,6a} = 11.2 Hz, ³J_{6b,5} = 6.6 Hz, H_{6b}), 3.87 (dt, 1H, ³J_{5,4} = 1.0 Hz, ³J_{5,6} = 6.6 Hz, H₅), 2.43 (t, 1H, ⁴J_{1''',1'} = 2.4 Hz, H_{1'''}), 2.06-1.89 (4s, 12H, CH₃CO₂)
**RMN ¹³C (CDCl₃,** 75 MHz) δ 170.13-169.29 (CH₃CO₂), 98.49 (C₁), 78.15 (C_{1"}), 75.40 (C_{1'''}), 70.68 (C₃ ou C₅), 70.64 (C₃ ou C₅), 68.36 (C₂), 66.88 (C₄), 61.07 (C₆), 55.72 (C_{1'}), 20.58-20.37 (CH₃CO₂)

### Composé 6 : β-D-galactopyranoside de propargyle de formule :

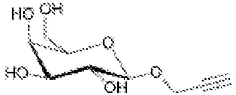

Le composé 5 (11,13 mmol, 4,3 g) est dissous dans du méthanol (2,47 mol, 100 mL). Une solution de sodium (10,0 mmol, 230 mg) dans du méthanol (2,47 mol, 100 mL) est ensuite ajoutée au milieu réactionnel. Le mélange est agité pendant 15 h à température ambiante. Une résine échangeuse d'ions IR-120 [H+] est ajoutée jusqu'à pH = 5-6. Le milieu réactionnel est filtré sur verre fritté et la résine est lavée avec du méthanol. Les solvants sont évaporés sous pression réduite et le résidu obtenu est séché une nuit au dessiccateur. Le composé 6 est obtenu avec un rendement de 91 % (2,2 g).
**Aspect :** solide blanc
**M :** 218,20 g.mol⁻¹
**Formule brute :** C₉H₁₄O₆
**Rf :** 0,28 (CH₂Cl₂ / MeOH : 80 / 20)
**Pf :** 94-96 °C
**[α]_{D}²⁰ :** -8,6° (c = 0,25, H₂O)
**ESI-MS (M** + **Na+) :** 241
**FT-IR (ATR en cm⁻¹) :** 3257 (_{UO-H}), 3234 2938-2874 (_{UC-H}), 1365-1294 (_{UC-H}), 1044- 1026 (_{UC-O})
**RMN ¹H (D₂O, 300MHz)** δ 4.59 (d, 1H, ³J_{1,2} = 7.8 Hz, H₁), 4.53 (dd, 1H, ²J_{1'a,1'b} = 15.9 Hz, ⁴J_{1'a,1'''} = 2.5 Hz, H_{1'a}), 4.46 (dd, 1H, ²J_{1'b,1'a} = 15.9 Hz, ⁴J_{1'b,1'''} = 2.5 Hz, H_{1'b}), 3.95 (dd, 1H, ³J_{5,4} = 0.3 Hz, ³J_{5,6} = 3.5 Hz, H₅), 3.78 (m, 3H, H₆ₐ, H_{6b}, H₄), 3.68 (dd, 1H, ³J_{3,2} = 9.9 Hz, ³J_{3,4} = 3.5 Hz, H₃), 3.55 (dd, 1H, ³J_{2,1} = 7.8 Hz, ³J_{2,3} = 9.9 Hz, H₂), 2.93 (t, 1H, ⁴J_{1''',1'} = 2.5 Hz, H_{1'''})
**RMN ¹³C (D₂O, 75MHz)** δ 101.13 (C₁), 78.99 (C_{1''}), 76.30 (C_{1'''}), 75.26 (C₄), 72.75 (C₃), 70.55 (C₂), 68.60 (C₅), 60.95 (C₆), 56.51 (C_{1'})

### Composé 8 : 1,2,3,4-tétra-O-acétyyl-L-rhamnopyranose de formule :

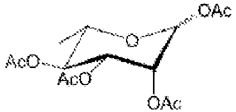

Le L-rhamnose (109,0 mmol, 20,0 g) et l'anhydride acétique (0,85 mol, 80 mL) sont mis en solution dans de la pyridine (0,99 mol, 80 mL). Le milieu réactionnel est agité pendant 15 h à température ambiante. Le solvant est évaporé sous pression réduite. Le résidu obtenu est dissous dans 250 mL d'acétate d'éthyle et lavé avec 2 fois 100 mL d'eau distillée, 2 fois 100 mL d'une solution saturée de NaHCO₃ et 1 fois 100 mL d'une solution saturée de NaCl. La phase organique est séchée sur du MgSO₄ puis filtrée sur verre fritté. Les solvants sont évaporés sous pression réduite puis co-évaporés avec 5 fois 100 mL de toluène. Le sirop obtenu est séché une nuit au dessiccateur. Le composé **8** est ainsi obtenu dans un mélange α / β : 86 / 14 et avec un rendement de 98 % (35,1 g).
**Aspect :** sirop incolore
**M :** 328,31 g.mol⁻¹
**Formule brute :** C₁₅H₂₀O₈
**Rf :** 0,58 (cyclohexane / AcOEt : 50 / 50)
**ESI-MS (M** + **Na+) :** 355
**FT-IR (ATR en cm⁻¹)** 2956 (_{UC-H}), 1744 (uc=o), 1368 (_{UC-O}), 1209 (_{UC-O})
**RMN ¹H (CDCl₃, 300MHz) diastéréoisomère α** : δ 5.96 (d, 1H, ³J_{1,2}= 1.9 Hz, H₁), 5.27 (dd, 1H, ³J_{3,2} = 3.5 Hz, ³J_{3,4} = 10.0 Hz, H₃), 5.20 (dd, 1H, ³J_{2,3} = 3.5 Hz, ³J_{2,1} = 1.9 Hz, H₂), 5.06 (dd, 1H, ³J_{4,3} = 10.0 Hz, ³J_{4,5} = 10.0 Hz, H₄), 3.89 (dd, 1H, ³J_{5,4} = 10.0 Hz, ³J_{5,6} = 6.3 Hz, H₅), 2.11-1.94 (4s, 12H, CH₃CO₂), 1.18 (d, 3H, ³J_{6,5} = 6.3 Hz, H₆)
**RMN ¹³C (CDC1₃, 75MHz) diastéréoisomère α** : δ 170.00-168.30 (CH₃CO₂), 90.63 (C₁), 70.46 (C₄), 68.78 (C₂, C₃ ou C₅), 68.70 (C₂, C₃ ou C₅), 68.64 (C₂, C₃ ou C₅), 20.85-20.62 (CH₃CO₂), 17,42 (C₆)

### Composé 9 : 2,3,4-tri-O-acétyl-α-L-rhamnopyranoside de propargyle de formule :

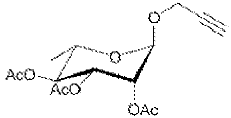

Le composé 8 (11,14 mmol, 3,8 g) est placé sous atmosphère inerte et dissous dans du dichlorométhane anhydre (0,78 mol, 50 mL). L'alcool propargylique (13,72 mmol, 0,81 mL) est additionné au milieu réactionnel ainsi que le diéthyle étherate de trifluorure de bore à 0 °C (45,74 mmol, 5,65 mL). Le mélange est agité pendant 15 h à température ambiante. Du carbonate de potassium (17,15 mmol, 2,37 g) est ajouté et on laisse agiter 30 minutes. Le milieu réactionnel est filtré sur verre fritté. Le filtrat jaune récupéré est concentré sous pression réduite, dilué avec 100 mL de dichlorométhane et lavé avec deux fois 100 mL d'eau distillée. Les phases aqueuses sont rassemblées et extraites avec deux fois 50 mL de dichlorométhane. Les phases organiques sont réunies et séchées sur du MgSO₄. Les solvants sont évaporés sous pression réduite. On récupère une huile brune qui est purifiée par chromatoflash automatisé (cyclohexane / AcOEt : 100 / 0 à 60 / 40 en 20 minutes). Le solide est séché une nuit au dessiccateur. Le composé 9 est ainsi obtenu avec un rendement de 79 % (2,88 g).
**Aspect :** solide blanc
**M :** 328,31 g.mol⁻¹
**Formule brute** : C₁₅H₂₀O₈
**Rf :** 0,65 (cyclohexane / AcOEt : 50 / 50)
**Pf :** 69-70 °C
**[α]_{D}²⁰ :** -84,6° (c=1, CHCl₃)
**ESI-MS (M** + **Na+) :** 351
**FT-IR (ATR en cm⁻¹) :** 3293 2988-2941 (_{UC-H}), 1742 (uc=o), 1220-1212 (_{UC-O}), 1054 (_{UC-O})
RMN **¹H (CDC1₃, 300MHz)** δ 5.33 (dd, 1H, ³J_{3,2} = 3.9 Hz, ³J_{3,4} = 9.7 Hz, H₃), 5.18 (dd, 1H, ³J_{2,3} = 3.9 Hz, ³J_{2,1} = 1.0 Hz, H₂), 4.99 (t, 1H, ³J_{4,3} = 9.7 Hz, H₄), 4.86 (dd, 1H, ³J_{1,2} = 1.0 Hz, H₁), 4.18 (d, 2H, ⁴J_{1',1'''} = 2.4 Hz, H_{1'}), 3.82 (dq, 1H, ³J_{5,4} = 9.7 Hz, ³J_{5,6} = 6.3 Hz, H₅), 2.43 (t, 1H, ⁴J_{1''',1'} = 2.4 Hz, H_{1'''}), 2.07-1.90 (3s, 9H, CH₃CO₂), 1.15 (d, 3H, ³J_{6,5} = 6.3 Hz, H₆)
**RMN ¹³C (CDC1₃, 75MHz)** δ 170.02-169.97 (CH₃CO₂), 96.11 (C₁), 78.27 (C_{1''}), 75.35 (C_{1'''}), 70.95 (C₄), 69.66 (C₂), 69.01 (C₃), 66.92 (C₅), 20.92-20.73 (CH₃CO₂), 17.32 (C₆)

### Composé 10 : α-L-rhamnopyranoside de propargyle de formule :

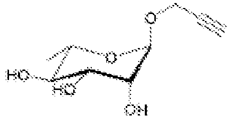

Le composé 9 (16,5 mmol, 5,41 g) est dissous dans du méthanol (2,47 mol, 100 mL). Une solution de sodium (10,0 mmol, 230 mg) dans du méthanol (2,47 mol, 100 mL) est ensuite ajoutée au milieu réactionnel. La solution est agitée pendant 15 h à température ambiante. Une résine échangeuse d'ions IR-120 [H⁺] est ajoutée jusqu'à pH = 5-6. Le mélange est filtré sur verre fritté et la résine est lavée avec du méthanol. Les solvants sont évaporés sous pression réduite et le résidu obtenu est séché une nuit au dessiccateur. Le composé 10 est obtenu avec un rendement de 90 % (3,0 g).
**Aspect** : solide blanc
**M :** 202,20 g.mol⁻¹
**Formule brute :** C₉H₁₄O₅
**Rf :** 0,77 (CH₂Cl₂ / MeOH : 80 / 20)
**Pf :** 105-107 °C
**[α]_{D}²⁰ :** -23,1°(c = 0,25, H₂O)
**ESI-MS (M** + **Na+) :** 225
**FT-IR (ATR en cm⁻¹) :** 3543-3336 (_{UO-H}), 3283 2946-2909 (_{UC-H}), 2119 (_{UC≡C}), 1446- 1228 (_{UO-H}), 1129-1048 (_{UC-O})
**RMN ¹H (D₂O, 300MHz)** δ 4.98 (d, 1H, ³J_{1,2} = 1.5 Hz, H₁), 4.37 (dd, 1H, ²J_{1'a,1'b} = 16.1 Hz, ⁴J_{1'a,1'''} = 2.4 Hz, H_{1'a}), 4.31 (dd, 1H, ²J_{1'b,1'a} = 16.1 Hz, ⁴J_{1'b,1'''} = 2.4 Hz, H_{1'b}), 3.96 (dd, 1H, ³J_{2,1} = 1.5 Hz, ³J_{2,3} = 3.4 Hz, H₂), 3.77 (dd, 1H, ³J_{3,2} = 3.4 Hz, ³J_{3,4} = 9.6 Hz, H₃), 3.74 (dd, 1H, ³J_{5,4} = 9.6 Hz, ³J_{5,6} = 6.3 Hz, H₅), 3.47 (dd, 1H, ³J_{4,3} = 9.6 Hz, ³J_{4,5} = 9.6 Hz, H₄), 2.94 (t, 1H, ⁴J_{1''',1'} = 2.4 Hz, H_{1'''}), 1.31 (d, 3H, ³J_{6,5} = 6.3 Hz, H₆)
**RMN ¹³C (D₂O, 75MHz)** δ 99.82 (C₁), 79.79 (C_{1''}), 77.09 (C_{1'''}), 72.85 (C₄), 71.10 (C₂), 70.94 (C₃), 70.02 (C₅), 55.64 (C_{1'}), 17.47 (C₆)

### Composé 17: S-(2,3,4-tri-O-acétyl-α-L-rhamnopyranosyl)thiouronium de formule:

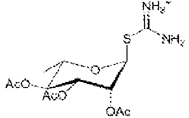

Le composé 8 (5,12 mmol, 1,68 g) et la thiourée (5,64 mmol, 0,43 g) sont placés sous atmosphère inerte et dissous dans de l'acétonitrile anhydre (0,19 mol, 10 mL). Le BF₃.Et₂O (10,76 mmol, 1,33 mL) est ajouté au milieu réactionnel. La solution est placée à reflux pendant 30 minutes. Le milieu réactionnel est refroidi à température ambiante et de la pyridine est ajoutée (10,76 mmol, 0,90 mL). Les solvants sont évaporés sous vide. Le sirop obtenu est dilué dans 15 mL d'isopropanol et le mélange est agité fortement à température ambiante pour cristalliser le complexe de pyridinium-bore. Le solide est filtré sur verre fritté et le filtrat concentré sous pression réduite. Le résidu obtenu est dissous dans 15 mL d'éthanol et le mélange est agité fortement à température ambiante. Le solide obtenu est filtré sur verre fritté, lavé avec de l'éthanol et séché une nuit au dessiccateur. Il est composé de 80 % en masse du composé **17** et de 20 % en masse du sel de pyridinium-trifluorure de bore. Le rendement est de 85 % (1,52 g).
**Aspect** : solide blanc
M : 349,38 g.mol-1
**Formule brute** : C₁₃H₂₁N₂O₇S
**ESI-MS (M** + **Na+)** : 371
**FT-IR (ATR en cm⁻¹)** 3389-3235 (_{UN-H}), 1734 (uc=o), 1659 (_{UN-H}), 1242-1066 (_{UC-O}) **RMN ¹H (C₂D₆SO, 300MHz)** δ 9.31 (s, 2H, NH₂), 9.25 (s, 2H, NH₂), 6,21 (d, 1H, ³J_{1,2} = 1,2 Hz, H₁), 5.37 (dd, 1H, ³J_{2,1} = 1.6 Hz, ³J_{2,3} = 2.9 Hz, H₂), 4.99 (m, 2H, H₃ et H₄), 4.51 (m, 1H, H₅), 2.14-1.96 (3s, 9H, CH₃CO₂), 1,19 (d, 3H, ³J_{6,5} = 6.2 Hz, H_{6b})
**RMN ¹³C (C₂D₆SO, 75MHz)** δ 169.72-169.36 (CH₃CO₂), 165.15 (SC(NH₂)₂), 81.67 (C₁), 69.59 (C₃ ou C₄), 69.12 (C₂ et C₅), 68.43 (C₃ ou C₄), 20.47-20.37 (CH₃CO₂), 17.11 (C₆)

### Composé 18 : 2,3,4-tri-O-acétyl-1-thio-α-L-rhamnopyranoside de propargyle de formule :

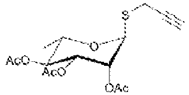

Le composé 17 (3,22 mmol, 1,12 g) est placé sous atmosphère inerte et dissous dans de l'acétonitrile anhydre (38,30 mmol, 20 mL). De la triéthylamine (9,97 mmol, 1,39 mL) est ajoutée suivie par du bromure de propargyle à 80 % dans le toluène (3,54 mmol, 0,39 mL). Le milieu réactionnel est agité pendant 1 h à température ambiante. Le solvant est évaporé sous vide et le sirop obtenu est dilué dans 30 mL de toluène. La phase organique est lavée avec 3 fois 30 mL d'eau distillée. Les phases aqueuses sont rassemblées et extraites avec 50 mL de toluène. Les phases organiques sont réunies et séchées sur du MgSO₄. Le solvant est évaporé sous pression réduite. Le sirop jaunâtre obtenu est purifié par chromatographie manuelle sur gel de silice avec un gradient d'éluant AcOEt / cyclohexane : 20 / 80 à 40 / 60. Le solide est séché une nuit au dessiccateur. Le composé 18 est obtenu avec un rendement de 73 % (0,81 g).
**Aspect :** sirop incolore
**M :** 344,38 g.mol-1
**Formule brute :** C₁₅H₂₀O₇S
**Rf :** 0,28 (cyclohexane / AcOEt : 75 / 25)
**[α]_{D}²⁰:** -161,8°(c=1, CHCl₃)
**ESI-MS (M + Na+) :** 367
**FT-IR (ATR en cm⁻¹) :** 3293 2985 (_{UC-H}), 1742-1939 (uc=o), 1213-1039 (_{UC-O})
**RMN ¹H (CDC1₃, 300MHz)** δ 5.39 (d, 1H, ³J_{1,2} = 1.3 Hz, H₁), 5.37 (dd, 1H, ³J_{2,3} = 3.2 Hz, ³J_{2,1} = 1.3 Hz, H₂), 5.18 (dd, 1H, ³J_{3,2} = 3.2 Hz, ³J_{3,4} = 10.0 Hz, H₃), 5.10 (dd, 1H, ³J_{4,3} = 10.0 Hz, ³J_{4,5} = 9.2 Hz, H₄), 4.18 (dq, 1H, ³J_{5,4} = 9.2 Hz, ³J_{5,6} = 6.2 Hz, H₅), 3.39 (dd, 1H, ²J_{1'a,1'b} = 16.8 Hz, ⁴J_{1'a,1'''} = 2.6 Hz, H_{1'a}), 3.22 (dd, 1H, ²J_{1'b,1'a} = 16.8 Hz, ⁴J_{1'b,1'''} = 2.6 Hz, H_{1'b}), 2.25 (t, 1H, ⁴J_{1''',1'} = 2.6 Hz, H_{1'''}), 2.15-1.96 (3s, 9H, CH₃CO₂), 1.23 (d, 3H, ³J_{6,5} = 6.2 Hz, H₆)
**RMN ¹³C (CDC1₃, 75MHz)** δ 169.86-169.79 (CH₃CO₂), 81.35 (C₁), 78.80 (C_{1"}), 71.92 (C_{1'''}), 71.06 (C₄), 70.79 (C₂), 69.54 (C₃), 67.45 (C₅), 20.87-20.61 (CH₃CO₂), 18.24 (C_{1'}), 17.31 (C₆)

### Composé 19 : 1-thio-α-L-rhamnopyranoside de propargyle de formule :

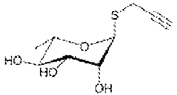

Le composé **18** (3,48 mmol, 1,20 g) est dissous dans du méthanol (1,23 mol, 50 mL). Une solution de sodium (5,0 mmol, 115 mg) dans du méthanol (1,23 mol, 50 mL) est ajoutée au milieu réactionnel. Le mélange est agité pendant 15 h à température ambiante. De la résine échangeuse d'ions IR-120 [H⁺] est ajouté jusqu'à pH =5-6. Le milieu réactionnel est filtré sur verre fritté et la résine est lavée au méthanol. Le solvant est évaporé sous pression réduite. Le sirop obtenu est séché une nuit au dessiccateur. Le composé 19 est ainsi obtenu avec un rendement de 88 % (0,67 g).
**Aspect :** sirop jaunâtre
**M :** 218,27 g.mol-1
**Formule brute :** C₆H₁₄O₄S
**Rf :** 0,70 (CH₂Cl₂ / MeOH : 75 / 25)
**[α]_{D}²⁰ :** -298,4° (c=1, MeOH)
**ESI-MS (M** + **Na+) :** 241
**FT-IR (ATR en cm⁻¹)** : 3385 (_{UO-H}), 3288 2976-2933 (_{UC-H}), 1058 (_{UC-O})
**RMN ¹H (D₂O, 300MHz)** δ 5.42 (s, 1H, H₁), 4.05 (dd, 1H, ³J_{2,3} = 3.5 Hz, ³J_{2,1} = 1.5 Hz, H₂), 4.00 (dq, 1H, ³J_{5,4} = 3.4 Hz, ³J_{5,6} = 6.3 Hz, H₅), 3.71 (dd, 1H, ³J_{3,2} = 3.5 Hz, ³J_{3,4} = 9.7 Hz, H₃), 3.47 (dd, 1H, ³J_{4,3} = 9.7 Hz, ³J_{4,5} = 3.4 Hz, H₄), 3.45 (dd, 1H, ²J_{1'a,1'b} = 17.1 Hz, ⁴J_{1'a,1'''} = 2.6 Hz, H_{1'a}), 3.34 (dd, 1H, ²J_{1'b,1'a} = 17.1 Hz, ⁴J_{1'b,1'''} = 2.6 Hz, H_{1'b}), 2.67 (t, 1H, ⁴J_{1''',1'} = 2.6 Hz, H_{1'''}), 1.29 (d, 3H, ³J_{6,5} = 6.3 Hz, H₆)
**RMN ¹³C (D₂O, 75MHz)** δ 84.65 (C₁), 80.83 (C_{1"}), 73.06 (C_{1'''}), 72.95 (C₄), 72.13 (C₂), 71.62 (C₃), 70.13 (C₅), 18.51 (C_{1'}), 17.33 (C₆)

### Composé 25 : 2,3,6-tri-O-benzoyl-β-D-galactopyranoside de propargyle de formule :

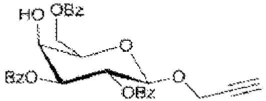

Le composé 6 (0,92 mmol, 200 mg) est dissous dans de la pyridine (30,9 mmol, 2,5 mL) et est refroidi à 0 °C. Du chlorure de benzoyle (3,30 mmol, 0,38 mL) est ensuite ajouté et la solution est agitée 15 h à température ambiante. La réaction est stoppée par ajout de 4 mL d'eau distillée. Le milieu réactionnel est dilué par 20 mL de toluène puis lavé avec successivement 20 mL d'une solution saturée de NaHCO₃, 20 mL d'une solution de HCI à 1M et enfin 20 mL d'eau distillée. La phase organique est séchée sur du MgSO₄ et les solvants sont évaporés sous pression réduite. Le résidu est purifié sur chromatoflash automatisé (cyclohexane / AcOEt : 100 / 0 à 80 / 20 en 30 minutes). Le solide est séché pendant une nuit au dessiccateur. Le composé 25 est obtenu avec un rendement de 70 % (342 mg).
**Aspect :** solide blanc
**M :** 530,52 g.mol⁻¹
**Formule brute :** C₃₀H₂₆O₉
**Rf :** 0,71 (cyclohexane / AcOEt : 50 / 50)
**Pf :** 78-79 °C
**[α]_{D}²⁰ :** 25,0° (c = 1, CHCl₃)
**ESI-MS (M+Na+) :** 553
**FT-IR (ATR en cm⁻¹) :** 3458 (_{UO-H}), 2971-2927 (_{UC-H}), 1722-1704 (uc=o), 1318-1261 (_{UC-O}), 1111-1072 (_{UC-O})
**RMN ¹H (CDCl₃,** 300 MHz) δ 8.05-7.96 (m, 6H, CH=CH), 7.60-7.30 (m, 9H, CH=CH), 5.82 (dd, 1H, ³J_{2,1} = 8.0 Hz, ³J_{2,3} = 10.3 Hz, H₂), 5.40 (dd, 1H, ³J_{3,2} = 10.3 Hz, ³J_{3,4} = 3.2 Hz, H₃), 5.04 (d, 1H, ³J_{1,2} = 8.0 Hz, H₁), 4.70 (dd, 1H, ²J_{6a,6b} = 11.4 Hz, ³J_{6a,5} = 6.4 Hz, H₆ₐ), 4.70 (dd, 1H, ²J_{6b,6a} = 11.4 Hz, ³J_{6b,5} = 6.4 Hz, H_{6b}), 4.47 (dd, 1H, ²J_{1'a,1'b} = 16.0 Hz, ⁴J_{1'a,1'''} = 2.3 Hz, H_{1'a}), 4.41 (d, 1H, ³J_{4,3} = 3.2 Hz, H₄), 4.37 (dd, 1H, ²J_{1'b,1'a} = 16.0 Hz, ⁴J_{1'b,1'''} = 2.3 Hz, H_{1'b}), 4.13 (t, 1H, ³J_{5,6} = 6.4 Hz, H₅), 2.95 (s, 1H, OH), 2.38 (t, 1H, ⁴J_{1''',1'} = 2.3 Hz, H_{1'''})
**RMN ¹³C (CDCl₃,** 75 MHz) δ 166.57-165.59 (PhCO₂), 133.56-133.21 (CH=CH), 129.98-128.36 (CH=CH), 98.83 (C₁), 78.44 (C_{1"}), 75.44 (C_{1'''}), 74.32 (C₃), 72.70 (C₅), 69.40 (C₂), 67.35 (C₄), 62.96 (C₆), 55.87 (C_{1'})

### Composé 27 : 4-S-(2,3,4-tri-O-acétyl-α-L-rhamnopyranosyl)-2,3,6-tri-O-benzoyl-4-thio-β-D-glucopyranoside de propargyle de formule :

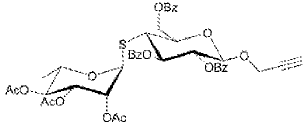

Le composé 25 (1,22 mmol, 650 mg) est placé sous atmosphère inerte et dissous dans du dichlorométhane anhydre (0,14 mol, 9 mL). Le milieu réactionnel est placé à-20 °C et de la pyridine (9,8 mmol, 0,80 mL) est ajoutée suivie par l'ajout de Tf₂O (2,5 mmol, 0,42 mL). Le mélange est mis sous agitation pendant 2 h en passant de -20 °C à 10 °C. La solution est diluée avec 20 mL de dichlorométhane et lavée avec successivement 30 mL d'une solution d'HCI à 1M, 30 mL d'une solution saturée de NaHCO₃ et 30 mL d'une solution saturée de NaCl. La phase organique est séchée sur du MgSO₄. On obtient un sirop jaune directement placé sous atmosphère inerte. De l'acétonitrile anhydre (0,19 mol, 10 mL), de la triéthylamine (4,4 mmol, 0,60 mL) et enfin le composé **17** (1,47 mmol, 647 mg) sont ajoutés. Le milieu réactionnel est agité pendant 2 h à température ambiante. Le solvant est évaporé sous pression réduite et le résidu obtenu est dilué par 30 mL de toluène. La phase organique est lavée avec 2 fois 30 mL d'eau distillée. Les phases aqueuses sont rassemblées et extraites avec 2 fois 30 mL de toluène. Les phases organiques sont réunies et séchées sur du MgSO₄. Le solvant est évaporé sous pression réduite. Le sirop est purifié sur chromatoflash automatisé (cyclohexane / AcOEt : 100 / 0 à 75 / 25 en 20 minutes). Le résidu est séché pendant une nuit au dessiccateur. Le composé 27 est obtenu avec un rendement de 60 % (599 mg).
**Aspect :** solide blanc
**M :** 818,84 g.mol⁻¹
**Formule brute :** C₄₂H₄₂O₁₅S
**Rf :** 0,61 (cyclohexane / AcOEt : 50 / 50)
**Pf :** 84-96 °C
**[a]_{D}²⁰ :** -22,8° (c = 1, CHCl₃)
**HRMS :** 841,2150 (841,2142 calculé pour C₄₂H₄₂O₁₅SNa)
**FT-IR (ATR en cm⁻¹)** : 3286 2972-2927 (_{UC-H}), 1722-1704 (uc=o), 1318-1261 (_{UC-O}), 1139-1028 (_{UC-O}), 718 (_{UC-O})
**RMN ¹H (CDCl₃, 600 MHz)** : 8.08 (m, 2H, CH=CH), 7.95 (m, 4H, CH=CH), 7.60 (m, 1H, CH=CH), 7.49 (m, 4H, CH=CH), 7.36 (m, 4H, CH=CH), 5.66 (dd, 1H, ³J_{3,2} = 9.5 Hz, ³J_{3,4} = 11.1 Hz, H_{Glc3}), 5.46 (dd, 1H, ³J_{2,1} = 8.0 Hz, ³J_{2,3} = 9.5 Hz, H_{Glc2}), 5.45 (d, 1H, ³J_{1,2} = 1.3 Hz, H_{Rha1}), 5.24 (dd, 1H, ³J_{2,1} = 1.3 Hz, ³J_{2,3}= 2.9 Hz, H_{Rha2}), 5.03 (d, 1H, ³J_{1,2} = 8.0 Hz, H_{Glc1}), 4.96 (dd, 1H, ³J_{3,4} = 10.0 Hz, ³J_{3,2} = 2.9 Hz, H_{Rha3}), 4.95 (d, 1H, J = 7.9 Hz, H_{Rha4}), 4.92 (dd, 1H, ²J_{6a,6b} = 12.1 Hz, ³J_{6a,5} = 2.3 Hz, H_{Glc6a}), 4.65 (dd, 1H, ²J_{6b,6a} = 12.1 Hz, ³J_{6b,5} = 4.4 Hz, H_{Glc6b}), 4.40 (dd, 1H, ²J_{1'a,1'b} = 16.0 Hz, ⁴J_{1'a,1'''} = 2.4 Hz, H_{Glc1'a}), 4.33 (dd, 1H, ²J_{1'b,1'a} = 16.0 Hz, ⁴J_{1'b,1'''} = 2.4 Hz, H_{Glc1'b}), 4.07 (ddd, 1H, ³J_{5,4} = 11.1 Hz, ³J_{5,6a} = 2.3 Hz, ³J_{5,6b} = 4.4 Hz, H_{Glc5}), 3.92 (dq, 1H, ³J_{5,4} = 2.0 Hz, ³J_{5,6} = 6.2 Hz, H_{Rha5}), 3.36 (t, 1H, ³J_{4,3} = 11.1 Hz, ³J_{4,5} = 11.1 Hz, H_{Glc4}), 2.39 (t, 1H, ⁴J_{1''',1'} = 2.4 Hz, H_{Glc1'''}), 1.99-1.89 (3s, 9H, CH₃CO₂), 0.96 (d, 3H, ³J_{6,5} = 6.2 Hz H_{Rha6})
**RMN ¹³C (CDCl₃, 150 MHz)** : 169.66-169.49 (CH₃CO₂), 165.76-165.23 (PhCO₂), 133.22-133.16 (CH=CH), 129.84-128.23 (CH=CH), 98.04 (C_{Glc1}), 81.12 (C_{Rha1}), 78.09 (C_{Glc1"}), 75.66 (C_{Glc1'''}), 74.64 (C_{Glc5}), 72.49 (C_{Glc2}), 71.18 (C_{Glc3}), 70.84 (C_{Rha2}), 70.53 (C_{Rha3} ou C_{Rha4}), 69.01 (C_{Rha3} ou C_{Rha4}), 67.77 (C_{Rha5}), 63.51 (C_{Glc6}), 55.72 (C_{Glc1'}), 46.06 (C_{Glc4}), 20.55-20.49 (CH₃CO₂), 17.02 (C_{Rha6})

### Composé 28 :[1-thio-α-L-rhamnopyranosyl-(1→4)]-β-D-alucopyranoside de propargyle de formule :

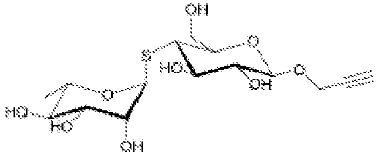

Le composé 27 (0,60 mmol, 484 mg) est dissous dans du méthanol (0,37 mol, 15 mL). Une solution de sodium (1,25 mmol, 29 mg) dans du méthanol (0,25 mol, 10 mL) est ajoutée au milieu réactionnel. La solution est agitée pendant 15 h à température ambiante. De la résine acide IR-120 [H+] est additionnée jusqu'à pH = 5-6. Le mélange est filtré sur verre fritté et la résine est lavée au méthanol. Le solvant est évaporé sous vide et le résidu purifié sur chromatoflash automatisé (CH₂Cl₂/ MeOH : 100 / 0 à 75 / 25 en 30 minutes). Le solide obtenu est séché une nuit au dessiccateur. On obtient le composé 28 avec un rendement de 88 % (197 mg).
**Aspect** : **solide blanc**
**M** : 380,41 g.mol⁻¹
**Formule brute** : C₁₅H₂₄O₉S
**Rf** : 0,48 (CH₂Cl₂/ MeOH : 75 / 25)
**Pf :** 47-69 °C
**[α]_{D}²⁰:** -166,6° (c = 1, MeOH)
**HRMS** : 403,1031 (403, 1039 calculé pour C₁₅H₂₄O₉SNa)
**FT-IR (ATR en cm⁻¹)** : 3398 (UO-H), 2972-2900 (UC-H), 1056 (UC-O)
**RMN ¹H (CDCl₃, 600 MHz)** : 5.31 (d, 1H, ³J_{1,2} = 1.2 Hz, H_{Rha1}), 4.63 (d, 1H, ³J_{1,2} = 8.1 Hz, H_{Glc1}), 4.50 (dd, 1H, ²J_{1'a,1'b} = 15.9 Hz, ⁴J_{1'a,1'"} = 2.3 Hz, H_{Glc1'a}), 4.46 (dd, 1H, ²J_{1'b,1'a} = 15.9 Hz, 4J_{1'b,1'"} = 2.3 Hz, H_{Glc1'b}), 4.13 (dq, 1H, ³J_{5,4}= 9.6 Hz, ³J_{5,6} = 6.3 Hz, H_{Rha5}), 4.11 (dd, 1H, ³J_{2,1} = 1.7 Hz, ³J_{2,3}= 3.3 Hz, H_{Rha2}), 4.08 (dd, 1H,²J_{6a,6b} = 12.2 Hz, ³J_{6a,5} = 2.0 Hz, H_{Glc6a}), 3.90 (dd, 1H, ²J_{6b,6a} = 12.2 Hz, ³J_{6b,5} = 5.2 Hz, H_{Glc6b}), 3.72 (dd, 1H, ³J_{3,4} = 9.6 Hz, ³J_{3,2} = 3.3 Hz, H_{Rha3}), 3.69 (ddd, 1H, ³J_{5,4} = 10.9 Hz, ³J_{5,6a} = 2.0 Hz, ³J_{5,6b} = 5.2 Hz, H_{Glc5}), 3.61 (dd, 1H, ³J_{3,2}= 9.1 Hz, ³J_{3,4} = 10.9 Hz, H_{Glc3}), 3.49 (t, 1H, ³J_{4,3} = 9.6 Hz, ³J_{4,5} = 9.6 Hz, H_{Rha4}), 3.34 (t, 1H, ³J_{2,3}= 8.5 Hz, ³J_{2,1} = 8.5 Hz, H_{Glc2}), 2.93 (t, 1H, ⁴J_{1'",1'} = 2.3 Hz, H_{Glc1'''}), 2.78 (t, 1H, ³J_{4,3} = 10.9 Hz, ³J_{4,5} = 10.9 Hz, H_{Glc4}), 1.30 (d, 3H, ³J_{6,5} = 6.3 Hz H_{Rha6})
**RMN ¹³C (CDCl₃, 150 MHz):** 100.38 (C_{Glc1}), 83.66 (C_{Rha1}), 78.93 (C_{Glc1"}), 76.49 (C_{Glc1'"}), 76.43 (C_{Glc5}), 74.16 (C_{Glc2}), 72.52 (C_{Glc3}, C_{Rha2} ou C_{Rha4}), 72.28 (C_{Glc3}, C_{Rha2} ou C_{Rha4}), 72.12 (C_{Glc3}, C_{Rha2} ou C_{Rha4}), 70.82 (C_{Rha3}), 69.68 (C_{Rha5}), 61.46 (C_{Glc6}), 56.54 (C_{Glc1'}), 48.02 (C_{Glc4}), 16.60 (C_{Rha6})

### Composé 54 :3,6-di-O-pivaloyl-β-D-glucopyranoside de propargyle de formule :

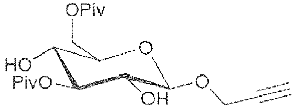

Une solution du composé 3 (4,58 mmol, 1 g) dans de la pyridine (98,9 mmol, 8 mL) est placée à 0 °C. Du chlorure de pivaloyle (11,45 mmol, 1,41 mL) est ensuite ajouté goutte à goutte. Le milieu réactionnel est agité durant 2 h 30 à O °C. Le solvant est évaporé sous pression réduite et le résidu obtenu est dissous dans 100 mL d'acétate d'éthyle. La phase organique est lavée avec successivement 100 mL d'une solution d'HCl diluée, 100 mL d'une solution saturée de NaHCO₃ et enfin 100 mL d'une solution saturée de NaCl. La phase organique est séchée sur du MgSO₄ et le solvant est évaporé sous vide. Le résidu est purifié par chromatographie manuelle sur gel de silice avec un gradient d'éluant : AcOEt / cyclohexane : 10 / 90 à 40 / 60. Le solide obtenu est séché une nuit au dessiccateur. On obtient le composé **54** avec un rendement de 53 % (0,94 g).
**Aspect** : Solide jaunâtre
**M :** 386,44 g.mol⁻¹
**Formule brute** : C₁₉H₃₀O₈
**Rf** : 0,61 (cyclohexane / AcOEt : 50/50)
**Pf** : 65-66 °C
**[α]_{D}²⁰** : -17,3° (c = 0,5, CHCl₃)
**ESI-MS (M** + **Na₊)** : 409
**FT-IR (ATR en cm⁻¹)** : 3475 (UO-H), 3280 (UC≡H), 2972-2874 (UC-H), 1716 (uc=o), 1283- 1036 (UC-O)
**RMN ¹H (CDCl₃, 300MHz)** δ 4.93 (t, 1H, ³J_{3,2} = 9.2 Hz, ³J_{3,4} = 9.2 Hz, H₃), 4.58 (d, 1H, ³J_{1,2} = 7.8 Hz, H₁), 4.42 (dd, 1H, ²J_{6a,6b} = 12.0 Hz, ³J_{6a,5} = 2.4 Hz, H₆ₐ), 4.40 (dd, 1H, ²J_{1'a,1'b} = 15.8 Hz, ⁴J_{1'a,1'"} = 2.4 Hz, H_{1'a}), 4.34 (dd, 1H, ²J_{1'b,1'a} = 15.8 Hz, ⁴J_{1'b,1'"} = 2,4 Hz, H_{1'b}), 4,27 (dd, 1H, ²J_{6b-6a} = 12,0 Hz, ³J_{6b-5} = 6,1 Hz, H_{6b}), 3,60 (ddd, 1H, ³J₅₋₆ₐ = 2,4 Hz, ³J_{5-6b} = 6,1 Hz, ³J₅₋₄ = 9.8 Hz, H₅), 3.50 (dd, 1H, ³J_{2,1} = 7.8 Hz, ³J_{2,3} = 9.2 Hz, H₂), 3.47 (dd, 1H, ³J_{4,3} = 9.2 Hz, ³J_{4,5} = 9.8 Hz, H₄), 2.48 (t, 1H,⁴J_{1''',1'} = 2.4 Hz, H_{1'"}), 1.23-1.20 (2s, 18H, (CH₃)₃CCO₂)
**RMN ¹³C (CDC1₃, 75MHz)** δ 180.25-178.90 ((CH₃)₃CCO₂), 100.31 (C₁), 78.46 (C_{1"}), 77.72 (C₃), 75.71 (C_{1'"}), 74.57 (C₅), 72.04 (C₂), 69.82 (C₄), 63.38 (C₆), 55.98 (C_{1'}), 39.15-39.00 ((CH₃)₃CCO₂), 27.26-27.18 ((CH₃)₃CCO₂)

### Composé 55 : [(2,3,4-tri-O-acétyl-α-L-rhamnopyranosyl)-(1→4)]-[(2,3,4-tri-O-acétyl-α-L-rhamnopyranosyl)-(1→2)1-3,6-di-O-pivaloyl-β-D-glucopyranoside de propargyle de formule :

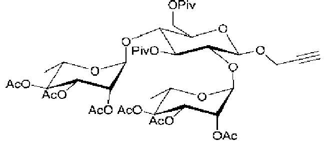

À du tamis moléculaire 4 Ǻ activé placé sous atmosphère, on ajoute le composé **54** (1,56 mmol, 600 mg) préalablement dissous dans 5 mL de dichlorométhane anhydre. Le milieu réactionnel est placé à -78 °C et du BF₃-Et₂O (7,02 mmol, 0,86 mL) est ajouté. La solution est agitée pendant 1 h à -78 °C. On ajoute enfin le composé **53** (4,68 mmol, 2,03 g) en solution dans 5 mL de dichlorométhane anhydre. Le mélange réactionnel est agité pendant 15 h à température ambiante. Le tamis moléculaire est filtré sur célite et lavé avec de l'acétate d'éthyle. Le filtrat est concentré sous pression réduite et le résidu obtenu est repris par 20 mL de chloroforme. La phase organique est lavée avec 20 mL d'une solution saturée de NaHCO₃ puis 20 mL d'une solution saturée de NaCl. Elle est ensuite séchée sur du MgSO₄ et le solvant est évaporé sous vide. Le solide jaunâtre obtenu est purifié en phase normale sur chromatoflash automatisé (cyclohexane / AcOEt : 100 / 0 à 0 / 100 en 30 minutes) ou par phase inverse sur chromatoflash automatisé (H₂O / MeOH : 70 / 30 à 0 / 100 en 20 minutes). Le solide obtenu est séché une nuit au dessiccateur. Le composé **55** est isolé avec un rendement de 71 % (1,03 g).
**Aspect** : solide blanc
**M** : 930,44 g.mol⁻¹
**Formule brute** : C₄₃H₆₂O₂₂
**Rf** : 0,60 (cyclohexane / AcOEt : 50/50)
**Pf** : 82-86 °C
**[α]_{D}²⁰ :** -48,4° (c = 1, CHCl₃)
**HRMS** : 953,3591 (953,3630 calculé pour C₄₃H₆₂O₂₂Na)
**FT-IR (ATR en cm⁻¹)** : 3445 (UO-H), 2977 (UC-H), 1745 (uc=o), 1220-1042 (UC-O)
**RMN ¹H (CDCl₃, 600 MHz)** : 5.26 (t, 1H, ³J_{3,4} = ³J_{3,2} = 7.4 Hz, H_{Glc3}), 5.23 (dd, 1H, ³J_{3,4} = 10.0 Hz, ³J_{3,2} = 3.4 Hz, H_{Rha3'}), 5.19 (d, 1H, ³J_{2,3} = 3.4 Hz, H_{Rha2'}), 5.18 (dd, 1H, ³J_{3,4}= 10,0 Hz, ³J_{3,2} = 3.2 Hz, H_{Rha3}), 5.05 (dd, 1H, ³J_{2,1} = 1.6 Hz, ³J_{2,3} = 3.2 Hz, H_{Rha2}), 5.04 (dd, 1H, ³J_{4,3}= 10.0 Hz, ³J_{4,5} = 10.0 Hz, H_{Rha4} ou H_{Rha4'}), 5.01 (dd, 1H, ³J_{4,3} = 10.0 Hz, ³J_{4,5} = 10.0 Hz, H_{Rha4} ou H_{Rha4'}), 4.87 (d, 1H, ³J_{1,2} = 1.6 Hz, H_{Rha1}), 4.81 (s, 1H, H_{Rha1'}), 4.74 (d, 1H, ³J_{1,2} = 7.0 Hz, H_{Glc1}), 4.48 (dd, 1H, ²J_{6a,6b} = 12.1 Hz, ³J_{6a,5} = 1.6 Hz, H_{Glc6a}), 4.36 (dd, 1H, ²J_{1'a,1'b}= 16.0 Hz, ⁴J_{1'a,1'''}= 2.3 Hz, H_{Glc1'a}), 4.33 (dd, 1H, ²J_{1'b,1'a}= 16.0 Hz, ⁴J_{1'b,1"'}= 2.3 Hz, H_{Glc1'b}), 4.26 (dd, 1H, ²J_{6b,6a}= 12.1 Hz, ³J_{6b,5} = 4.5 Hz, H_{Glc6b}), 4.21 (dq, 1H, ³J_{5,4} = 10.0 Hz, ³J_{5,6} = 6.3 Hz, H_{Rha5} ou H_{Rha5'}), 3.91 (dq, 1H, ³J_{5,4} = 10.0 Hz, ³J_{5,6} = 6.3 Hz, H_{Rha5} ou H_{Rha5'}), 3.78 (m, 2H, H_{Glc4} et H_{Glc5}), 3.59 (t, 1H, ³J_{2,3} = 7.4 Hz, ³J_{2,1} = 7.4 Hz, H_{Glc2}), 2,49 (t, 1H, ⁴J_{1''',1'} = 2.3 Hz, H_{Glc1'''}), 2.11-1.94 (6s, 18H, CH₃CO₂), 1.22 (s, 9H, (CH₃)₃CCO₂), 1.19 (d, 3H, ³J_{6,5}= 6.3 Hz, H_{Rha6} ou H_{Rha6'}), 1.17 (s, 9H, (CH₃)₃CCO₂), 1.15 (d, 3H, ³J_{6,5} = 6.3 Hz H_{Rha6} ou H_{Rha6'})
**RMN ¹³C (CDCl₃, 150 MHz) :** 177.74-176.45 ((CH₃)₃CCO₂), 170.02-169.53 (CH₃CO₂), 98.11 (C_{Rha1'}), 98.16 (C_{Glc1}), 97.42 (C_{Rha1}), 78.02 (C_{Glc'''}), 77.79 (C_{Glc2}), 76.56 (C_{Glc4} ou C_{Glc5}), 75.74 (C_{Glc1"}), 75.08 (C_{Glc3}), 72.32 (C_{Glc4} ou C_{Glc5}), 70.94 (C_{Rha4} ou C_{Rha4'}), 70.53 (C_{Rha4} ou C_{Rha4'}), 69.98 (C_{Rha2} ou C_{Rha2'}), 69.61 (C_{Rha2} ou C_{Rha2'}), 69.05 (C_{Rha3} ou C_{Rha3'}), 68.69 (C_{Rha3} ou C_{Rha3'}), 67.97 (C_{Rha5} ou C_{Rha5'}), 66.81 (C_{Rha5} ou C_{Rha5'}), 62.40 (C_{Glc6}), 55.51 (C_{Glc1'}), 38.88-38.81 ((CH₃)₃CCO₂), 27.15-26.86 ((CH₃)₃CCO₂), 20.80-20.67 (CH₃CO₂), 17.16-17.09 (C_{Rha6} et C_{Rha6'})

### Composé 56 : [(α-L-rhamnopyranosyl)-(1→4)]-[(α-L-rhamnopyranosyl)-(1→2)]-β-D-glucopyranoside de propargyle de formule :

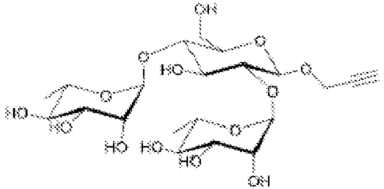

Le composé **55** (0,81 mmol, 749 mg) est dissous dans du méthanol (0,61 mol, 25 mL). Une solution de sodium (25 mmol, 575 mg) dans du méthanol (0,61 mol, 25 mL) est ajoutée. Le mélange réactionnel est agité pendant 15 h à température ambiante. De la résine acide IR-120 [H+] est additionnée jusqu'à obtention d'un pH = 5-6. La résine est filtrée sur verre fritté et lavée au méthanol. Le solvant est évaporé sous pression réduite et le résidu obtenu est purifié en phase inverse sur chromatoflash automatisé (H₂O / MeOH : 100 / 0 à 0 / 100 en 20 minutes). Le produit est séché une nuit au dessiccateur. Le composé **56** est isolé avec un rendement de 87 % (360 mg).
**Aspect** : solide blanc
**M :** 510,49 g.mol⁻¹
**Formule brute** : C₂₁H₃₄O₁₄
**Rf** : 0,17 (CH₂Cl₂/ MeOH : 75/25)
**Pf** : 56-66 °C
**[α]_{D}²⁰**: -98,7° (c = 1, MeOH)
**HRMS** : 533,1857 (533,1846 calculé pour C₂₁H₃₄O₁₄Na)
**FT-IR (ATR en cm⁻¹)** : 3390 (UO-H), 2931 (UC-H), 1128-1041 (UC-O)
**RMN ¹H (CDCl₃, 600 MHz) :** 5.03 (d, 1H, ³J_{1,2}= 1.5 Hz, H_{Rha1}), 4.85 (d, 1H, ³J_{1,2}= 1.4 Hz, H_{Rha1'}), 4.71 (d, 1H, ³j_{1,2} = 7.9 Hz, H_{Glc1}), 4.50 (dd, 1H, ²J_{1a',1'b}= 15.8 Hz, ⁴J_{1'a,1'"} = 2.3 Hz, H_{Glc1'a}), 5.46 (dd, 1H, ²J_{1'b,1'a}= 15.8 Hz, ⁴J_{1'b,1'"}= 2.3 Hz, H_{Glc1'b}), 4.03 (m, 4H, H_{Rha2}, H_{Rha2'}, H_{Rha5} et H_{Rha5'}), 3.89 (dd, 1H, ²J_{6a,6b}= 12.4 Hz, ³J_{6a,5}= 2.2 Hz, H_{Glc6a}), 3.77 (t, 1H, ³J_{3,2} = ³J_{3,4} = 9.8 Hz, H_{Rha3}), 3.76 (t, 1H, ³J_{3,2} = ³J_{3,4} = 9.8 Hz, H_{Rha3'}) 3.74 (m, 2H, H_{Glc3} et H_{Glc6b}), 3.59 (t, 1H, ³J_{4,3} = 9.5 Hz, ³J_{4,5} = 9.5 Hz, H_{Glc4}), 3.51 (ddd, 1H, ³J_{5,4} = 9.5 Hz, ³J_{5,6a} = 2.2 Hz, ³J_{5,6b} = 4.6 Hz, H_{Glc5}), 3.47 (t, 1H, ³J_{4,3} = ³J_{4,5} = 9.8 Hz, H_{Rha4}), 3.46 (t, 1H, ³J_{4,3} = ³J_{4,3} = 9.8 Hz, H_{Rha4'}), 3.38 (dd, 1H, ³J_{2,1} = 7,9 Hz, ³J_{2,3} = 9.0 Hz, H_{Glc2}), 2.94 (t, 1H, ⁴J_{1''',1'} = 2.3 Hz, H_{Glc1'''}), 1.29 (d, 3H, ³J_{6,5} = 6.3 Hz, H_{Rha6}), 1.26 (d, 3H, ³J_{6,5} = 6,3 Hz, H_{Rha6'})
**RMN ¹³C (CDCl₃, 150 MHz) :** 101.78 (C_{Rha1}), 101.02 (C_{Rha1'}), 99.34 (C_{Glc1}), 80.19 (C_{Glc2}), 78.84 (C_{Glc1"}), 77.05 (C_{Glc4}) 76.59 (C_{Glc1"'}), 75.15 (C_{Glc5}) 74.93 (C_{Glc3}) 72.08 (C_{Rha4} ou C_{Rha4'}), 72.00 (C_{Rha4} ou C_{Rha4'}), 70.46 (C_{Rha2}, C_{Rha2'}, C_{Rha3} ou C_{Rha3'}), 70.33 (C_{Rha2}, C_{Rha2'}, C_{Rha3} ou C_{Rha3'}), 70.29 (C_{Rha2}, C_{Rha2'}, C_{Rha3} ou C_{Rha3'}), 70.27 (C_{Rha2}, C_{Rha2'}, C_{Rha3} ou C_{Rha3'}), 69.12 (C_{Rha5} ou C_{Rha5'}), 69.04 (C_{Rha5} ou C_{Rha5'}), 60.18 (C_{Glc6}), 56.62 (C_{Glc1'}), 16.76-16.57 (C_{Rha6} et C_{Rha6'})

### Composé 66 : 3-azidopropan-1-ol de formule :

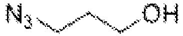

Le 3-chloropropan-1-ol (0,11 mol, 10 g) est dissous dans de l'eau distillée (0,81 mol, 45 mL). De l'azoture de sodium (0,21 mmol, 13,78 g) est ajouté et le milieu réactionnel est agité pendant 15 h à 80 °C. Le milieu réactionnel est lavé avec trois fois 50 mL de diéthyl éther. Les phases organiques sont réunies et séchées sur du MgSO₄. Le solvant est évaporé sous pression réduite à froid (15 °C), car le produit est volatile. Le composé **66** est obtenu avec un rendement de 99 % (11,0 g).
**Aspect** : liquide incolore
**M :** 101,11 g.mol-1
**Formule brute** : C₃H₇N₃O
**Rf** : 0,47 (cyclohexane / AcOEt : 50 / 50)
**ESI-MS (M + Na₊)** : 124
**FT-IR (ATR en cm⁻¹)** 3332 (UO-H), 2946 (UC-H), 2883 (UC-H), 2089 (UN≡N), 1258 (UC-O), 1045 (UC-N)
**RMN ¹H (CDCl₃, 300 MHz)** δ 3.60 (s, 1H, OH), 3.47 (t, 2H, ³J = 6.4 Hz, CH₂OH), 3.21 (t, 2H, ³J = 6.4 Hz, CH₂N₃), 1.61 (qt, 2H, ³J = 6,4 Hz, ³J = 6,4 Hz, CH₂CH₂CH₂)
**RMN ¹³C (CDCl₃, 75 MHz)** δ 58.63 (CH₂OH), 47.74 (CH₂N₃), 30.94 (CH₂CH₂CH₂)

### Composé 67 : p-toluènesulfonate de 3-azidopropyl de formule :

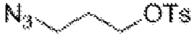

Le composé **66** (0,11 mol, 11,5 g) est mis en solution dans du dichlorométhane (1,72 mol, 110 mL). Du DMAP (23,0 mmol, 2,78 g) puis de la triéthylamine (0,17 mol, 23,8 mL) sont ensuite ajoutés. Le milieu réactionnel est placé à 0 °C. Une solution de chlorure de tosyle (0,17 mol, 32,41 g) dans du dichlorométhane (0,94 mol, 60 mL) est ajoutée à la réaction. Le mélange réactionnel est agité pendant 15 h en passant de 0 °C à température ambiante. Le milieu réactionnel est dilué avec 200 mL de dichlorométhane et lavé avec successivement 200 mL d'une solution saturée de NaHCO₃, 200 mL d'une solution à 10 % en volume de HCI et 200 mL d'une solution saturée de NaCl. La phase organique est séchée avec du MgSO₄ et les solvants sont évaporés sous pression réduite. Le résidu est purifié par chromatographie manuelle sur gel de silice suivant un gradient d'éluant : cyclohexane/ Et₂O : 80 / 20 à 60 / 40. Le composé **67** est obtenu avec un rendement de 76 % (21,4 g).
**Aspect** : cristal incolore
**M** : 255,29 g.mol-1
**Formule brute** : C₁₀H₂₃N₃O₃S
**Rf** : 0,57 (cyclohexane / Et₂O : 50 / 50)
**Pf** : 28-29 °C
**ESI-MS (M + Na₊)** : 278
**FT-IR (ATR en cm⁻¹)** 2957 (UC-H), 2095 (UN≡N), 1598 (UC=N), 1356 (USO2), 1188 (USO2), 1172 (UC-O), 662 (USO2)
**RMN ¹H (CDCl₃, 300MHz)** δ 7.80 (d, 2H, ³J = 8.0 Hz, CH=CH), 7,38 (d, 2H, ³J = 8.0 Hz, CH=CH), 4.12 (t, 2H, ³J = 6,3 Hz, CH₂OSO₂), 3.38 (t, 2H, ³J = 6.3 Hz, CH₂N₃), 2.45 (s, 3H, CH₃), 1.89 (q^{t}, 2H, ³J = 6,3Hz, CH₂CH₂CH₂)
**RMN ¹³C (CDCl₃, 75MHz)** δ 144.86 (CSO₃), 132.42 (CCH₃), 129.73 (CH=CH), 127.54 (CH=CH), 67.01 (CH₂OSO₂), 46.99 (CH₂N₃), 28.06 (CH₂CH₂CH₂), 21.24 (CCH₃)

### Composé 75 : 3-O-(3-azidopropyl)solanidine de formule :

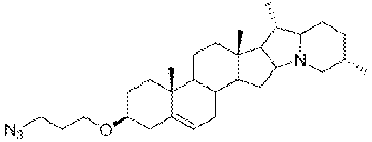

La solanidine (0,25 mmol, 100 mg) est placée sous atmosphère inerte et dissoute dans du THF anhydre (49,3 mmol, 4mL). Du NaH à 95 % (0,50 mmol, 13 mg) est ensuite ajouté et le mélange réactionnel est agité pendant 30 minutes à température ambiante. On ajoute ensuite du composé **67** (1,26 mmol, 0,32 g) et la solution est agitée pendant 24 h à 60 °C. Les solvants sont évaporés sous pression réduite puis le résidu est dissous dans du chloroforme et filtré sur célite. Après concentration du filtrat, le résidu obtenu est purifié par CPC (conditions décrites ci-dessus). Un mélange de solanidine et du composé 75 est isolé. Le produit 75 est recristallisé dans l'acétonitrile avec un rendement de 71 % (85 mg).
**Aspect** : solide incolore
**M** : 469,75 g.mol⁻¹
**Formule brute** : C₃₀H₅₁N₃O
**Rf** : 0.59 (toluène 100 %)
**Pf** : 42-48 °C
**[α]_{D}**²⁰: -14.4° (c = 0.25, CHCl₃)
**HRMS** : 492,3919 (492,3930 calculé pour C₃₀H₅₁N₃ONa)
**FT-IR (ATR en cm⁻¹)** : 2991 (U_{C-H}), 2216 (U_{N≡N}), 1648 (U_{C=N}), 1547 (u_{C=C}), 1026 (U_{C-O})
**RMN ¹H (CDCl₃, 300MHz)** δ 5.34 (dd, 1H, ³J_{6,7a} = 1.7 Hz, ³J_{6,7b} = 3.3 Hz, H₆), 3.54 (t, 2H, ³J_{3"',3"} = 6.2 Hz, H_{3'''}), 3.39 (t, 2H, ³J_{3',3"} = 6.7 Hz, H_{3'}), 3.13 (m, 1H, H₃), 2.35 (ddd, 1H, ²J_{4a,4b} = 13.2 Hz, ³J_{4a,3} = 2.1 Hz, J = 4.7 Hz, H₄ₐ), 2.18 (dd, 1H, ²J_{4b,4a} = 13.2 Hz, ³J_{4b,3} = 2.1 Hz, H_{4b}), 2.04-1.76 (m, 4H), 1.82 (dt, 2H, ³J_{3",3'} = 6.7 Hz, ³J_{3",3'"} = 6.2 Hz, H_{3"}), 1.62-1.02 (m, 22H), 0.99 (s, 3H, H₁₉), 0.91 (d, 3H, ³J_{21,20} = 6.5 Hz, H₂₁), 0.86 (d, 3H, ³J_{27,25} = 6.6 Hz, H₂₇), 0,85 (d, 3H, ³J_{26,25} = 6.6 Hz, H₂₆), 0.67 (s, 3H, H₁₈)
**RMN ¹³C (CDCl₃, 75MHz)** δ 140.94 (C₅), 121.73 (C₆), 79.37 (C₃), 64.54 (C_{3'}), 56.89 (C₁₄), 56.31 (C₁₇), 50.32 (C₉), 48.68 (C_{3'''}), 42.44 (C₁₃), 39.91 (C₁₂), 39.64 (C₂₄), 39.21 (C₄), 37.35 (C₁), 36.99 (C₁₀), 36.33 (C₂₂), 35.92 (C₂₀), 32.06 (C₇), 32.01 (C₈), 29.73 (C_{3"}), 28.50 (C₂), 28.36 (C₁₆), 28.12 (C₂₅), 24.41 (C₁₅), 23.98 (C₂₃), 22.94 (C₂₇), 22.69 (C₂₆), 21.20 (C₁₁), 19.48 (C₁₉), 18.84 (C₂₁), 11.97 (C₁₈)

### 1-3. Préparations des composés selon l'invention

### Composé 100 : 3-O-{3-[4-(β-D-glucopyranosyloxyméthyl)-1,2,3-triazol-1-yl]propyl}solanidine de formule :

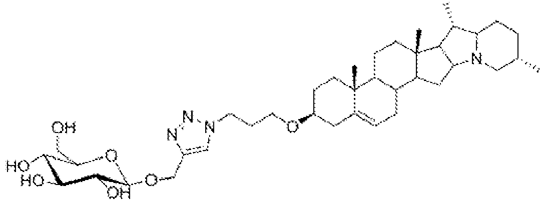

Le composé **75** (0,08 mmol, 40 mg) et le composé **3** (0,10 mmol, 22 mg) sont dissous dans 3,75 mL d'un mélange de 1,4-dioxane / H₂O : 4 / 1 en volume. Du sulfate de cuivre (II) (0,12 mmol, 19 mg) et de l'ascorbate de sodium (0,23 mmol, 47 mg) sont ensuite ajoutés. Le milieu réactionnel est agité pendant 24 h à 80 °C. Le mélange est filtré sur célite et le filtrat récupéré est concentré sous pression réduite. Le résidu est purifié par CPC. Le composé **100** est obtenu avec un rendement de 48 % (27 mg).
**Aspect** : huile jaune
**M** : 698,93 g.mol-1
**Formule brute** : C₃₉H₆₂N₄O₇
**Rf** : 0,29 (CHCl₃/ MeOH : 80 / 20)
**[α]_{D}²⁰ :** -19,2° (c = 0,1, MeOH)
**HRMS** : 699,4722 (699,4697 calculé pour C₆₆H₆₃N₄O₇)
**FT-IR (ATR en cm⁻¹)** : 3393 (UO-H), 2925-2854 (UC-H), 1076-1036 (UC-O)
**RMN ¹H (pyridine-d₅, 600 MHz) :** 8.17 (s, 1H, H_{Glc1"'}), 5.38 (d, 1H, ²J_{1'a,1'b}= 12.3 Hz, H_{Glc1'a}), 5.36 (s, 1H, H₆), 5.17 (d, 1H, ²J_{1'b,1'a}= 12.3 Hz, H_{Glc1'b}), 5.07 (d, 1H, ³J_{1,2} = 7.6 Hz, H_{Glc1}), 4.57 (dd, 1H, ²J_{6a,6b}= 12.0 Hz, ³J_{6a,5} = 2.4 Hz, H_{Glc6a}), 4.52 (t, 2H, J = 9.8 Hz, H_{3"'}), 4.38 (dd, 1H, ²J_{6b},₆ₐ= 12.0 Hz, ³J_{6b},₅ = 5.3 Hz, H_{Glc6b}), 4.26 (m, 2H, H_{Glc3} et H_{Glc4}), 4.09 (m, 1H, H_{Glc2}), 3.98 (m, 1H, H_{Glc5}), 3.60 (s, 1H, H₂₆ₐ), 3.44 (m, 3H, H_{3'} et H₁₆), 3.08 (m, 1H, H₃), 2.56-1.84 (m, 6H, H₄, H_{3"}, H₂₂ et H_{26b}), 1.72-1.10 (m, 24H), 0.93 (s, 3H, H₁₉), 0.92 (d, 3H, ³J_{21,20} = 13.1 Hz, H₂₁), 0.91 (m, 1H), 0.75 (d, 3H, ³J_{27,26} = 13.1 Hz, H₂₇)
**RMN ¹³C (pyridine-d₅**, **150 MHz)** : 145.63 (C_{Glc1"}), 141.39 (C₅), 124.71 (C_{Glc1'"}), 121.68 (C₆), 104.46 (C_{Glc1}), 79.53 (C₃), 78.95 (C_{Glc3}, C_{Glc4} ou C_{Glc5}), 78.89 (C_{Glc3}, C_{Glc4} ou C_{Glc5}), 75.87 (C₂₂), 75.50 (C_{Glc2}), 72.00 (C_{Glc3}, C_{Glc4} ou C_{Glc5}), 70.86 (C₁₆), 64.56 (C_{3'}), 63.48 (C_{Glc1'}), 63.07 (C_{Glc6}), 61.16 (C₁₇), 59.08 (C₂₆), 57.94 (C₁₄), 50.44 (C₉), 47.82 (C_{3'''}), 40.91 (C₁₃), 40.54 (C₁₂), 39.76 (C₄), 37.60 (C₁), 37.50 (C₁₀), 36.90 (C₂₀), 32.54 (C₂₄), 31.79 (C₁₅), 31.56 (C_{3"}), 31.40 (C₂₅), 30.29 (C₂₃), 29.04 (C₈), 28.57 (C₇), 27.35 (C₂), 21.42 (C₁₁), 19.66 (C₁₉), 18.88 (C₂₇), 16.87 (C₁₈), 16.35 (C₂₁)

### Composé 101 : 3-O-{3-[4-(β-D-galactopyranosyloxyméthyl-1,2,3-triazol-1-yl]propyl}solanidine de formule :

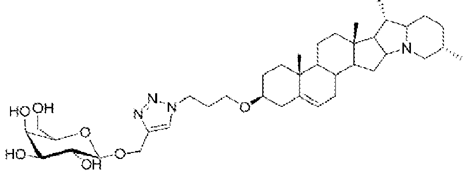

Le composé **75** (0,08 mmol, 40 mg) et le composé **6** (0,10 mmol, 22 mg) sont dissous dans 3,75 mL d'un mélange de 1,4-dioxane / H₂O : 4 / 1 en volume. Du sulfate de cuivre (II) (0,12 mmol, 19 mg) et de l'ascorbate de sodium (0,23 mmol, 47 mg) sont ensuite additionnés. Le milieu réactionnel est agité pendant 24 h à 80 °C. Le mélange est filtré sur célite et le filtrat récupéré est concentré sous pression réduite. Le résidu est purifié par CPC. Le composé **101** est obtenu avec un rendement de 55 % (31 mg).
**Aspect** : huile jaune
**M** : 698,93 g.mol⁻¹
**Formule brute** : C₃₉H₆₂N₄O₇
**Rf** : 0,23 (CHCl₃/ MeOH : 80 / 20)
**[α]_{D}²⁰** : -16,7° (c = 0,1, MeOH)
**HRMS** : 699,4711 (699,4697 calculé pour C₃₉H₆₃N₄O₇)
**FT-IR (ATR en cm⁻¹)** : 3378 (UO-H), 2934-2866 (UC-H), 1092-1041 (UC-O)
**RMN ¹H (pyridine-ds, 600 MHz)** : 8.11 (s, 1H, H_{Gal1'''}), 5,42 (d, J = 5.8 Hz, 1H, H₆), 5.41 (d, 1H, ²J_{1'a,1'b}= 12.3 Hz, H_{Gal1'a}), 5.19 (d, 1H, ²J_{1'b,1'a} = 12.3 Hz, H_{Gal1'b}), 5.00 (d, 1H, ³J_{1,2}= 7.7 Hz, H_{Gal1}), 4.58 (d, 1H, ³J_{4,3} = 3.0 Hz, H_{Gal4}), 4.50 (m, 5H, H_{3'"}, H_{Gal2}, H_{Gal6a} et H_{Gal6b}), 4.19 (dd, 1H, ³J_{3,4} = 3.0 Hz, ³J_{3,2} = 9.4 Hz, H_{Gal3}), 4.11 (t, 1H, J = 5.9 Hz, H_{Gal5}), 3.43 (dq, 2H, J = 6.0 Hz, J = 9.7 Hz, H_{3'}), 3.17 (dq, 1H, J = 11.3 Hz, J = 6.6 Hz, H₃), 2.93 (dd, 1H, J = 10.3 Hz, J = 2.5 Hz, H₂₆ₐ), 2.67 (m, 1H, H₁₆), 2.48 (dd, 1H, J = 13.1 Hz, J = 2.5 Hz, H₄ₐ), 2.32 (t, 1H, J = 11.3 Hz, H_{4b}), 2.12 (q^{t}, 2H, J = 6.6 Hz, H_{3"}), 2.04 (m, 1H), 1.92 (m, 1H), 1.81-1.38 (m, 18H), 1.26 (q, 1H, J = 13.0 Hz, H_{23b}), 1.14 (m, 3H), 1.03-0.93 (m, 3H), 0.99 (s, 3H, H₁₉), 0.98 (d, 3H, ³J_{21,20} = 5.9 Hz, H₂₁), 0.96 (s, 3H, H₁₈), 0.84 (d, 3H, ³J_{27,26} = 6.4 Hz, H₂₇), 0.82 (m, 1H, H_{24b})
**RMN ¹³C (pyridine-ds, 150 MHz) :** 145.78 (C_{Gal1"}), 141.40 (C₅), 124.19 (C_{Gal1"'}), 122.19 (C₆), 105.06 (C_{Gal1}), 79.64 (C₃), 77.55 (C_{Gal5}), 75.75 (C_{Gal3}), 75.13 (C₂₂), 72.90 (C_{Gal2}), 70.67 (C_{Gal4}), 69.62 (C₁₆), 64.62 (C_{3'}), 63.72 (C₁₇), 63.40 (C_{Gal1'}), 62.86 (C_{Gal6}), 60.64 (C₂₆), 58.14 (C₁₄), 50.87 (C₉), 47.85 (C_{3"'}), 40.94 (C₁₃), 40.45 (C₁₂), 39.92 (C₄), 37.76 (C₁), 37.55 (C₁₀), 37.28 (C₂₀), 34.00 (C₂₄), 32.77 (C₈), 32.32 (C₇), 31.90 (C₁₅), 31.70 (C₂₅), 31.64 (C_{3"}), 29.97 (C₂₃), 29.13 (C₂), 21.63 (C₁₁), 20.05 (C₂₇), 19.87 (C₁₉), 18.90 (C₂₁), 17.39 (C₁₈)

### Composé 102 : 3-O-{3-[4-(α-L-rhamnopyranosyloxyméthyl)-1,2,3-triazol-1-yl]propyl}solanidine de formule :

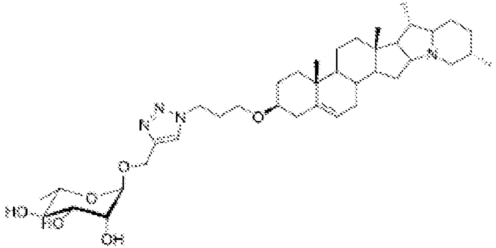

Le composé **75** (0,08 mmol, 40 mg) et le composé **10** (0,10 mmol, 20 mg) sont dissous dans 3,75 mL d'un mélange de 1,4-dioxane / H₂O : 4 / 1 en volume. Du sulfate de cuivre (II) (0,12 mmol, 19 mg) et de l'ascorbate de sodium (0,23 mmol, 47 mg) sont ensuite ajoutés. Le milieu réactionnel est agité pendant 24 h à 80 °C. Le mélange est filtré sur célite et le filtrat récupéré est concentré sous pression réduite. Le résidu est purifié par CPC. Le composé **102** est obtenu avec un rendement de 52 % (28 mg).
**Aspect** : huile jaune
**M** : 682,93 g.mol⁻¹
**Formule brute** : C₃₉H₆₂N₄O₆
**Rf** : 0,34 (CHCl₃ / MeOH : 80 / 20)
**[α]_{D}²⁰ :** -33,3° (c = 0,1, MeOH)
**HRMS** : 683,4735 (683,4748 calculé pour C₃₉H₆₃N₄O₆)
**FT-IR (ATR en cm⁻¹)** : 3379 (UO-H), 2955-2854 (UC-H), 1076-1011 (UC-O)
**RMN ¹H (pyridine-d₅, 600 MHz)** : 8.09 (s, 1H, H_{Rha1'"}), 5.52 (s, 1H, H_{Rha1}), 5,43 (d, J = 2.8 Hz, 1H, H₆), 5.19 (d, 1H, ²J_{1'a,1'b} = 12.1 Hz, H_{Rha1'a}), 4.97 (d, 1H, ²J_{1'b,1'a} = 12.1 Hz, H_{Rha1'b}), 4.57 (m, 3H, H_{3"'} et H_{Rha2}), 4.51 (d, 1H, J = 6.5 Hz, H_{Rha3} ou H_{Rha4}), 4.31 (m, 2H, H_{Rha5} et H_{Rha3} ou H_{Rha4}), 3.42 (m, 2H, H_{3'}), 3.17 (m, 1H, H₃), 2.96 (d, 1H, J = 7.6 Hz, H₂₆ₐ), 2.71 (m, 1H, H₁₆), 2.49 (d, 1H, J = 12.8 Hz, H₄ₐ), 2.36 (t, 1H, J = 11.8 Hz, H_{4b}), 2.16 (m, 2H, H_{3"}), 2.04 (d, 1H, J = 8.3 Hz), 1.94 (d, 1H, J = 11.9 Hz), 1.81-1.27 (m, 12H), 1.14 (m, 2H), 0.99 (m, 9H, H₁₈, H₁₉ et H₂₁), 0.91 (m, 4H), 0.84 (d, 3H, ³J_{27,26}= 10.8 Hz, H₂₇)
**RMN ¹³C (pyridine-ds, 150 MHz) :** 145.30 (C_{Rha1"}), 141.41 (C₅), 124.21 (C_{Rha1'"}), 122.18 (C₆), 101.39 (C_{Rha1}), 79.67 (C₃), 75.18 (C₂₂), 74.35 (C_{Rha3}, C_{Rha4} ou C_{Rha5}), 73.13 (C_{Rha3}, C_{Rha4} ou C_{Rha5}), 72.59 (C_{Rha2}), 70.47 (C_{Rha3}, C_{Rha4} ou C_{Rha5}), 69.68 (C₁₆), 64.65 (C_{3'}), 62.10 (C₁₇), 61.07 (C_{Gal1'}), 60.60 (C₂₆), 58.15 (C₁₄), 50.87 (C₉), 47.94 (C_{3'"}), 40.95 (C₁₃), 40.47 (C₁₂), 39.94 (C₄), 37.76 (C₁), 37.57 (C₁₀), 37.28 (C₂₀), 32.78 (C₈), 32.30 (C₇), 31.77 (C₂₅), 31.65 (C_{3"}), 31.15 (C₁₅ ou C₂₄), 30.74 (C₁₅ ou C₂₄), 30.37 (C₂₃), 29.13 (C₂), 21.63 (C₁₁), 20.02 (C₂₇), 19.95 (C_{Rha6}), 19.87 (C₁₉), 19.03 (C₂₁), 17.39 (C₁₈)

### Composé 103 : 3-O-{3-[4-(α-L-rhamnopyranosylthiométhyl)-1,2,3-triazol-1-yl]propyl}solanidine de formule :

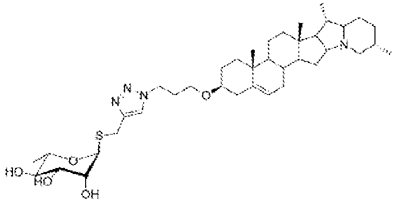

Le composé **75** (0,08 mmol, 40 mg) et le composé **19** (0,10 mmol, 22 mg) sont dissous dans 3,75 mL d'un mélange de 1,4-dioxane / H₂O : 4 / 1 en volume. Du sulfate de cuivre (II) (0,12 mmol, 19 mg) et de l'ascorbate de sodium (0,23 mmol, 47 mg) sont ensuite additionnés. Le milieu réactionnel est agité pendant 24 h à 80 °C. Le mélange est filtré sur célite et le filtrat récupéré est concentré sous pression réduite. Le résidu est purifié par CPC. Le composé 103 est obtenu avec un rendement de 63 % (pureté non confirmée par RMN).
**Aspect** : sirop brun
**M :** 699,00 g.mol-1
**Formule brute** : C₃₉H₆₂N₄O₅S
**[α]_{D}** ²⁰ : + 3 °(c = 0,1, MeOH)
**HRMS** : 699,4515 (699,4519 calculé pour C₃₉H₆₃N₄O₅S)
**FT-IR (ATR en cm⁻¹)** : 3383 (UO-H), 2926 (UC-H), 1122-1010 (UC-O)

### Composé 104 : 3-O-(3-{4-[1-thio-α-L-rhamnopyranosyl-(1→4)-β-D-glucopyranosyloxyméthyl]-1,2,3-triazol-1-yl}propyl)solanidine de formule :

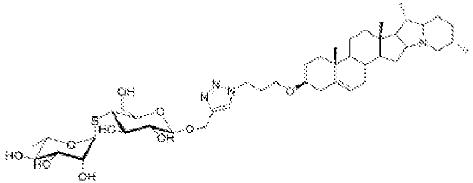

Le composé **75** (0,08 mmol, 40 mg) et le composé **28** (0,10 mmol, 38 mg) sont dissous dans 3,75 mL d'un mélange de 1,4-dioxane / H₂O : 4 / 1 en volume. Du sulfate de cuivre (II) (0,12 mmol, 19 mg) et de l'ascorbate de sodium (0,23 mmol, 47 mg) sont ensuite ajoutés. Le milieu réactionnel est agité pendant 24 h à 80 °C. Le mélange est filtré sur célite et le filtrat récupéré est concentré sous pression réduite. Le résidu est purifié par CPC. Le composé **104** est obtenu avec un rendement de 59 % (pureté non confirmée par RMN).
**Aspect** : sirop brun
**M** : 861,14 g.mol⁻¹
**Formule brute** : C₄₅H₇₂N₄O₁₀S
**[α]_{D}²⁰** : -38,2 ° (c = 0,1, MeOH)
**HRMS** : 861,5039 (861,5047 calculé pour C₄₅H₇₃N₄O₁₀S)
**FT-IR (ATR en cm⁻¹)** : 3339 (UO-H), 2935-2877 (UC-H), 1056 (UC-O)

### Composé 105 : 3-O-[3-(4-{[(α-L-rhamnopyranosyl)-(1→4)]-[(α-L-rhamnopyranosyl)-(1→2)]-β-D-glucopyranosyloxyméthyl}-1,2,3-triazol-1-yl)propyl]solanidine de formule :

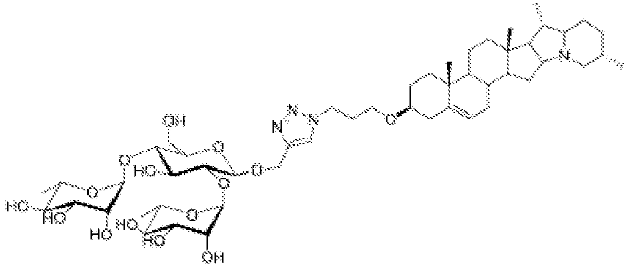

Le composé **75** (0,08 mmol, 40 mg) et le composé **56** (0,10 mmol, 51 mg) sont dissous dans 3,75 mL d'un mélange de 1,4-dioxane / H₂O : 4 / 1 en volume. Du sulfate de cuivre (II) (0,12 mmol, 19 mg) et de l'ascorbate de sodium (0,23 mmol, 47 mg) sont ensuite ajoutés. Le milieu réactionnel est agité pendant 24 h à 80 °C. Le mélange est filtré sur célite et le filtrat récupéré est concentré sous pression réduite. Le résidu est purifié par CPC. Le composé **105** est obtenu avec un rendement de 67 % (pureté non confirmée par RMN).
**Aspect** : sirop brun
**M** : 991,21 g.mol⁻¹
**Formule brute** : C₅₁H₈₂N₄O₁₅
**[α]_{D}²⁰** : -19,5 ° (c = 0,1, MeOH)
**HRMS** : 991,5820 (991,5855 calculé pour C₅₁H₈₃N₄O₁₅)
**FT-IR (ATR en cm⁻¹)** : 3347 (UO-H), 2931 (UC-H), 1126-980 (UC-O)

### Exemples 2 Tests biologiques 2-1. Insectes

La population de *Macrosiphum euphorbiae* a été initiée à partir d'une femelle aptère fournie par le Laboratoire de Biologie Fonctionnelle, Insectes et Interactions de l'INRA / INSA de Villeurbanne en 2004. Elle a été prélevée en 1995 sur aubergine dans la région Rhône-Alpes. Les pucerons de *Macrosiphum euphorbiae* ont été élevés sur plantes de pommes de terre de variété Désirée dans une salle à 20 ± 1 °C, 60 ± 5 % d'humidité relative et sous une photopériode de 16 heures de lumière et de 8 heures d'obscurité.

### 2-2. Dispositif de suivi physiologique

Pour les tests sur larve de *Macrosiphum euphorbiae,* des adultes ont été isolés et placés dans des cages de PVC sur milieu artificiel pendant une journée afin de synchroniser leur descendance. Les néonates (40 par expérience), âgés de moins de 24 heures, sont placés dans des cages en PVC contenant du milieu artificiel complémenté de 0,002, 0,02 ou 0,2 mM du composé actif ou sans complément pour l'expérience témoin.

Pour les tests sur *Macrosiphum euphorbiae* adulte, des néonates (50 par expérience) issus de la synchronisation sont élevés sur feuilles de plantes de pommes de terre de variété Désirée, dans des boîtes de Pétri pendant 10 jours. Une fois le stade adulte atteint, ils sont placés dans des cages en PVC contenant du milieu artificiel complémenté de 0,002, 0,02 ou 0,2 mM du composé actif ou sans complément pour l'expérience témoin.

Les individus ont été suivis tous les deux jours sur une période de 16 jours. À chaque relevé, le milieu artificiel est renouvelé. Chaque cage est constituée de cinq pucerons.

### 2-3. Milieu artificiel

Un régime standard a été utilisé comme support pour effectuer le suivi physiologique. Sa composition a été décrite par Febvay & al. (J. Zool. 1988, 66(11), 2449-2453) puis modifiée selon les conditions de Down & al. (J. Insect Physiol. 1996, 42 (11-12), 1035-1045) comme le montre le tableau 2. Le milieu est supplémenté du composé actif et placé entre deux couches de Parafilm® en conditions stériles.

**Tableau 2 : mode opératoire pour l'obtention du milieu artificiel adapté à Macrosiphum Euphorbiae**

| **Vitamines** | **mg** |
|---|---|
| Acide 4-aminobenzoique | 100,00 |
| Acide ascorbique | 1000,00 |
| Biotine | 1,00 |
| CaCl₂ | 50,00 |
| Chlorure de choline | 500,00 |
| Acide folique | 10,00 |
| Myo-inosirol | 420,00 |
| Acide nicotonique | 100,00 |
| Pyridoxine | 25,00 |
| Thiamine | 25,00 |
| | |

| **Acide aminés** | **mg** |
|---|---|
| Alanine | 178,70 |
| β-D-alanine | 6,22 |
| Arginine | 244,90 |
| Asparagine | 298,50 |
| Acide aspartique | 88,25 |
| Cystéine | 29,59 |
| Acide glutamique | 149,30 |
| Glutamine | 445,60 |
| Glycine | 166,50 |
| Histidine | 136,00 |
| Isoleucine | 164,70 |
| Leucine | 231,50 |
| Lysine | 351,00 |
| Methionine | |
| Ornithine | 9,41 |
| Phénylalanine | 293,00 |
| Proline | 129,30 |
| Sérine | 124,20 |
| Thréonine | 127,10 |
| Tryptophane | 42,75 |
| Tyrosine | 38,63 |
| Valine | 190,80 |

| | |
|---|---|
| **Traces de métaux** | **mg** |
| CuSO₄, 5H₂O | 0,47 |
| FeCl₃, 6H₂O | 4,45 |
| MnCl₂, 4H₂O | 0,65 |
| NaCl | 2,54 |
| ZnCl₂ | 0,83 |

| **Sucre** | **g** |
|---|---|
| Saccharose | 20 |
| | |

| **Autres** | **mg** |
|---|---|
| CaCl₂ | 3,00 |
| Acide citrique | 5,80 |
| Benzoate de Cholestéryle | 2,50 |
| MgSO₄, 7H₂O | 242,00 |
| | |

| **Préparation des vitamines:** | |
|---|---|
| 1 - Peser les vitamines et mettre dans un bécher. | |
| 2 - - Ajouter 150 mL d'eau stérile. | |
| 3 - Ajouter 5mg de Riboflavine (dissoute dans 1 mL d'eau stérile en chauffant à 50 °C). | |
| 4 - Ajuster le volume à 200 mL. | |
| 5 - Aliquoter par 10 mL et stocker à -20 °C. | |
| | |

| **Préparation du milieu artificiel** | |
|---|---|
| 1 - Peser les acides aminés, le sucre, les métaux et les autres. | |
| 2 - Ajouter 40 mL d'eau stérile et 20 mL du mélange de vitamines. | |
| 3 - Dissoudre pendant 2-3 heures avec agitateur magnétique. | |
| 4 - Ajuster le pH à 7 en ajoutant goutte à goutte une solution de KOH à 1M (0,56 g dans 10 mL d' eau). | |
| 5 - Ajouter 250 mg de KH₂PO₄. | |
| 6 - Ajuster à 100 mL et à pH 7,5. | |

### 2-4. Analyses statistiques

Les analyses statistiques ont été réalisées à l'aide du logiciel Statistica 10 (StatSoft®). Pour comparer la survie des pucerons par rapport au témoin, un test χ2 de Pearson (α < 0,05) a été effectué. Pour comparer les composés en fonction de leur concentration et de leur nature, un test PLSD de Fisher a été réalisé (α < 0,05). La fécondité journalière a aussi été étudiée par un test PLSD de Fisher (α < 0,05).

### 2-5. Effets sur la survie larvaire

Les tests biologiques ont été effectués sur des néonates de *Macrosiphum euphorbiae,* âgés de moins de 24 heures. Ils sont placés sur milieu artificiel contenant la chaconine, la solanine, la solanidine, **100, 101** ou **102.**

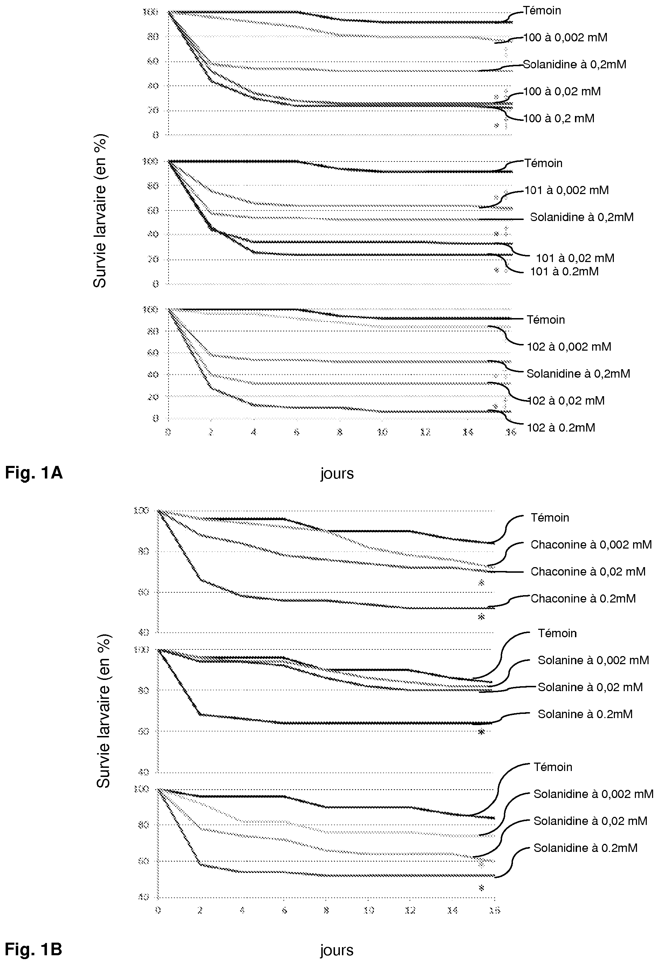

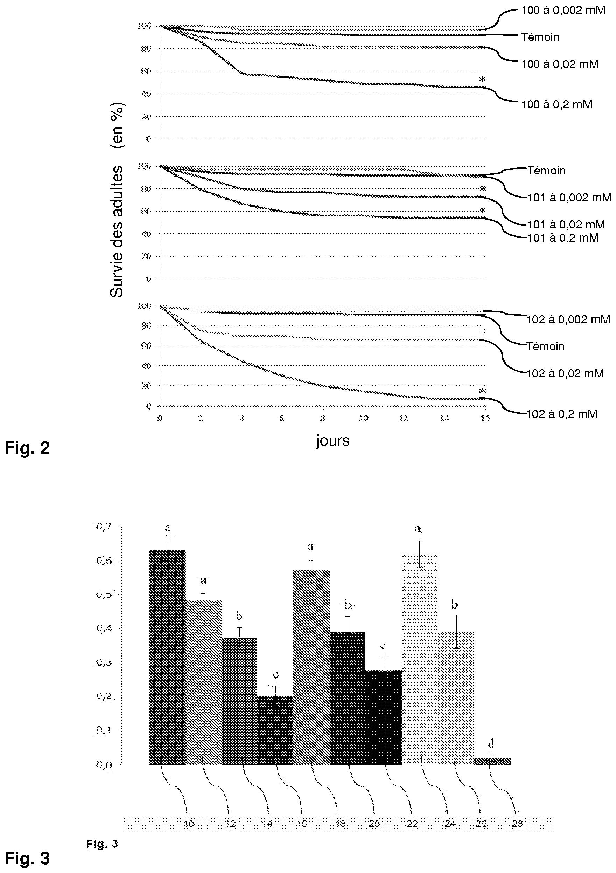

Les figures 2A et 2B présentent la variation de la mortalité dans le temps. La Figure 1A montre les effets sur la survie larvaire des composés 100, 101 et 102 selon l'invention. Les résultats ont été analysés par un test χ² de Pearson dans lequel α = 0,05, * signifie qu'il existe une différence significative par rapport au témoin. La Figure 1B montre les effets sur la survie larvaire de la chaconine, de la solanine et de la solanidine. Les résultats ont été analysés par un test χ² de Pearson dans lequel α = 0,05, * signifie qu'il existe une différence significative par rapport au témoin.

Selon ces résultats, l'activité aphicide des glycoalcaloïdes de synthèse est marquée par rapport au témoin. De plus, elle l'est aussi par rapport à la solanidine à 0,2 mM qui montre le meilleur résultat pour les molécules naturelles. De manière générale, les glycoalcaloïdes de synthèse ont une activité plus faible à 0,002 mM que la solanidine à 0,2 mM (p < 0,001 pour les composés **100** et **102** et p = 0,006 pour le composé **101**). Si la concentration de **100, 101** et **102** est augmentée à 0,02 mM, le taux de survivants est de 26, 33 et 32 % respectivement. À cette concentration, les propriétés aphicides sur les larves deviennent plus importantes qu'avec la solanidine. Cette observation est encore plus marquée pour les concentrations de 0,2 mM où la survie larvaire est de 22, 24 et 6 % pour **100, 101** et **102** respectivement (p <0,001).

Les résultats de ce test après 16 jours de traitement sont donnés dans le tableau 3 suivant. Les valeurs sont exprimées en pourcentage. Bien entendu, plus la valeur est faible, plus les composés sont puissants. Comme le montre le tableau 3, les composés selon l'invention ont des valeurs très faibles par rapport aux glycoalcaloïdes naturels. Par conséquent, des effets aphicides très puissants des composés selon l'invention par rapport aux glycoalcaloïdes naturels ont été démontrés.

**Tableau 3: pourcentage de survie larvaire pour chaque expérience, après 16 jours de traitement. Les valeurs suivies d'une même lettre indiquent qu'il n'existe pas de différence significative d'après le test de Pearson avec correction de Bonferroni (α = 0,005) ou d'après le test de PLSD de Fisher (α = 0,005).**

| | 0,002mM | 0,02mM | 0,2mM |
|---|---|---|---|
| 100 | 76a | 26b | 22c |
| 101 | 62a | 33b | 24c |
| 102 | 84a | 32b | 6d |
| Chaconine | 72a | 70a | 52b |
| Solanine | 82a | 80a | 64c |
| Solanidine | 74a | 60d | 52b |

### 2-6. Effets sur la survie adulte

Les tests biologiques des composés **100, 101** et **102** sur *Macrosiphum euphorbiae* adulte sont présentés en Figure 2, et sont comparés à une expérience témoin.

Le composé 100 ne possède pas d'activité significative par rapport au témoin à une concentration de 0,002 mM et de 0,02 mM. Cependant, un effet notable est observé à 0,2 mM (p < 0,001) avec un taux de mortalité de 54 %. En ce qui concerne le composé 101, une augmentation de la concentration à 0,02 et 0,2 mM permet d'observer une chute du taux de survie de *Macrosiphum euphorbiae* adulte (p = 0,006 et p < 0,001 respectivement). Enfin, le composé 102 n'a pas d'activité aphicide significative à la plus faible concentration, mais, tout comme le composé 101, une augmentation de la concentration du composé à 0,02 et 0,2 mM permet d'observer un effet marqué sur la survie des adultes (p < 0,001 pour les deux expériences).

Les résultats de ce test après 16 jours de traitement sont donnés dans le tableau 4 suivant. Les valeurs sont exprimées en pourcentage. Comme le montre le tableau 4, les composés selon l'invention ont des valeurs très faibles par rapport au témoin.

**Tableau 4 : pourcentage de survie des adultes après 16 jours de traitement. Les valeurs suivies d'une même lettre indiquent qu'il n'existe pas de différence significative d'après le test de PLSD de Fisher (α = 0,005).**

| | 0,002mM | 0,02mM | 0,2mM |
|---|---|---|---|
| 100 | 97a | 81b | 46c |
| 101 | 90a | 73b | 54c |
| 102 | 95a | 67b | 7d |

### 2-7. Effets sur la reproduction

Le relevé du nombre de larves issues de la parthénogenèse, associé à un traitement des données relevées, permet d'accéder à la fécondité journalière par puceron. Les résultats sont présentés sur la Figure 3 dans laquelle le numéro 10 représente le témoin, les numéros de référence 12, 14 et 16 représentent des résultats du composé 100 à des concentrations respectivement de 0,002, 0,02, et 0,2mM, les numéros de référence 18, 20 et 22 représentent des résultats du composé 101 à des concentrations respectivement de 0,002, 0,02, et 0,2mM, les numéros de référence 24, 26, et 28 représentent des résultats du composé 102 à des concentrations respectivement de 0,002, 0,02, et 0,2mM.

Comme pour le taux de survie, la présence des composés selon l'invention dans le milieu artificiel peut avoir un impact sur la reproduction. En comparaison avec l'expérience témoin, les composés **100, 101 et 102** réduisent la reproduction de manière significative à des concentrations de 0,02 et 0,2 mM (p < 0,001).

### Conclusion

Comme il a été décrit en détail ci-dessus, les composés selon l'invention ont les avantages suivants :
- Les modifications structurelles apportées par la synthèse permettent d'augmenter la toxicité des glycoalcaloïdes notamment sur *Macrosiphum euphorbiae* larve ou adulte.
- Les composés selon l'invention pourraient être utilisés en tant qu'insecticides contre d'autres espèces ravageuses de la pomme de terre, mais aussi contre des insectes non inféodés à cette plante.
- Contrairement aux glycoalcaloïdes naturels dans lesquels un trisaccharide est indispensable, une seule unité saccharidique serait suffisante pour engendrer une activité aphicide avec les composés selon l'invention.
- Les composés selon l'invention présentent une structure relativement simple et facile à synthétiser.
- Les composés selon l'invention possèdent des propriétés insecticides (le comportement de l'insecte n'est pas affecté, mais le composé agit sur les paramètres démographiques comme la survie et la reproduction). - Les composés selon l'invention peuvent être obtenus à partir de la chaconine et de la solanine, eux même obtenus à partir des coproduits de l'industrie de la pomme de terre, ce qui permet de les valoriser.
- Enfin, les composés selon l'invention possèdent d'autres activités possibles, comme des propriétés bactéricides, fongicides, nématicides, antivirales ou antitumorales.

## Revendications

1. Composé de formule (I) : 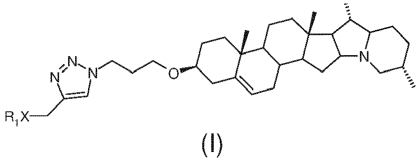 dans lequel :
- X est un atome d'oxygène ou un atome de soufre ; et
- R₁ représente un monosaccharide, un disaccharide, un thio-disaccharide, un trisaccharide ou un thio-trisaccharide, chaque unité saccharidique correspondant à un hexose quelconque et/ou à un pentose quelconque.

2. Composé de formule (I) selon la revendication 1 **caractérisé en ce que** ladite unité saccharidique est un hexose.

3. Composé de formule (I) selon les revendications 1 ou 2, **caractérisé en ce que** R₁ est choisi parmi le groupe consistant en un groupe glucosyle, un groupe galactosyle et un groupe rhamnosyle.

4. Composé de formule (I) selon l'une quelconque des revendications 1 à 3, **caractérisé en ce que** X est un atome d'oxygène.

5. Composé de formule (I) selon l'une quelconque des revendications 1 à 3, **caractérisé en ce que** X est un atome de soufre.

6. Composé de formule (II) :

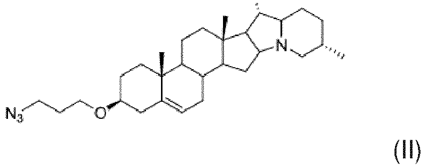

7. Utilisation non thérapeutique du composé de formule (I) selon l'une quelconque des revendications 1 à 5 en tant qu'insecticide.

8. Procédé d'obtention du composé de formule (I) selon l'une quelconque des revendications 1 à 5, dans lequel :
a) on fournit un composé de formule (II) : 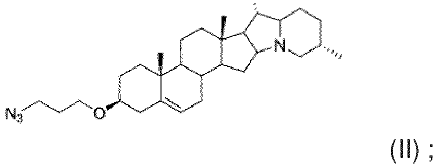 et
b) on fait réagir, en présence, ou non, d'un catalyseur, le composé de formule (II) avec un composé de formule (III) :

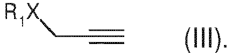

9. Procédé d'obtention du composé de formule (I) selon la revendication 8, dans lequel :
c) on fournit ledit composé de formule (IV) : 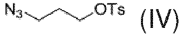 dans lequel Ts est un groupe tosyle ;
d) on fournit un composé de formule (V) : 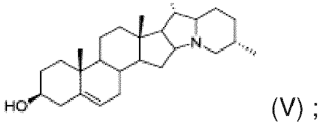 et
e) on fait réagir le composé de formule (V) avec le composé de formule (IV) pour obtenir le composé de formule (II).

10. Procédé d'obtention du composé de formule (I) selon les revendications 8 ou 9, **caractérisé en ce que** ledit catalyseur est choisi parmi des composés de cuivre ou de ruthénium.

11. Composition comprenant du composé de formule (I) selon l'une quelconque des revendications 1 à 5.

## Patentansprüche

1. Verbindung der Formel (I): 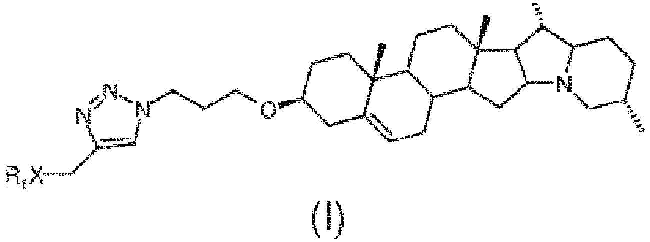 worin
- X ein Sauerstoffatom oder ein Schwefelatom ist; und
- R₁ ein Monosaccharid, ein Disaccharid, ein Thiodisaccharid, ein Trisaccharid oder ein Thiotrisaccharid darstellt, wobei jede Saccharideinheit einer beliebigen Hexose und / oder einer beliebigen Pentose entspricht.

2. Verbindung der Formel (I) gemäß Anspruch 1, **dadurch gekennzeichnet, dass** die Saccharideinheit eine Hexose ist.

3. Verbindung der Formel (I) gemäß den Ansprüchen 1 oder 2, **dadurch gekennzeichnet, dass** R₁ aus der Gruppe, bestehend aus einer Glucosylgruppe, einer Galactosylgruppe und einer Rhamnosylgruppe, ausgewählt ist.

4. Verbindung der Formel (I) gemäß einem der Ansprüche 1 bis 3, **dadurch gekennzeichnet, dass** X ein Sauerstoffatom ist.

5. Verbindung der Formel (I) gemäß einem der Ansprüche 1 bis 3, **dadurch gekennzeichnet, dass** X ein Schwefelatom ist.

6. Verbindung der Formel (II):

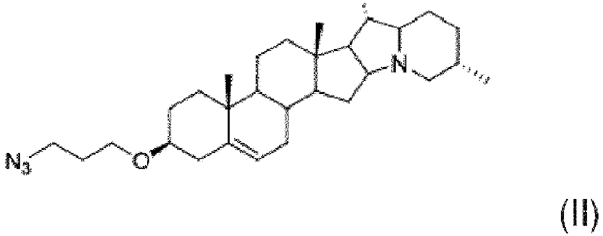

7. Nicht-therapeutische Verwendung der Verbindung der Formel (I) gemäß einem der Ansprüche 1 bis 5 als Insektizid.

8. Verfahren zum Erhalten der Verbindung der Formel (I) gemäß einem der Ansprüche 1 bis 5, wobei:
a) eine Verbindung der Formel (II) bereitgestellt wird: 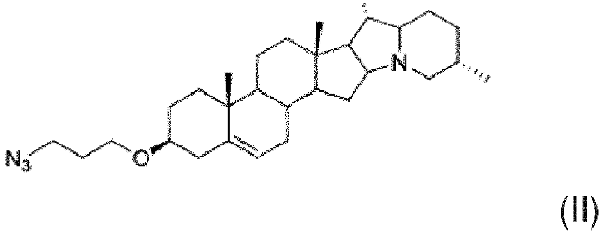 und
b) die Verbindung der Formel (II) in Gegenwart oder Abwesenheit eines Katalysators mit einer Verbindung der Formel (III) umgesetzt wird:

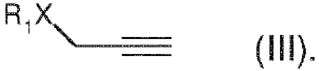

9. Verfahren zum Erhalten der Verbindung der Formel (I) gemäß Anspruch 8, wobei:
c) die Verbindung der Formel (IV) bereitgestellt wird: 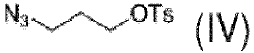 worin Ts eine Tosylgruppe ist;
d) eine Verbindung der Formel (V) bereitgestellt wird: 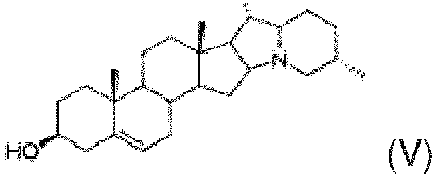 und
e) die Verbindung der Formel (V) mit der Verbindung der Formel (IV) umgesetzt wird, um die Verbindung der Formel (II) zu erhalten.

10. Verfahren zum Erhalten der Verbindung der Formel (I) gemäß den Ansprüchen 8 oder 9, **gekennzeichnet dadurch, dass** der Katalysator aus Verbindungen von Kupfer oder Ruthenium ausgewählt ist.

11. Zusammensetzung umfassend die Verbindung der Formel (I) gemäß einem der Ansprüche 1 bis 5.

## Claims

1. A compound of formula (I): 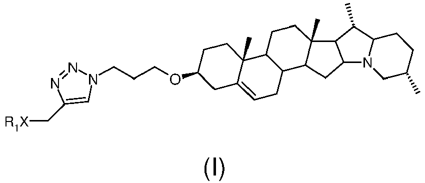 wherein:
- X is an oxygen atom or a sulfur atom; and
- R₁ represents a monosaccharide, a disaccharide, a thiodisaccharide, a trisaccharide or a thiotrisaccharide, each saccharide unit corresponding to any hexose and/or any pentose.

2. The compound of formula (I) as claimed in claim 1, **characterized in that** said saccharide unit is a hexose.

3. The compound of formula (I) as claimed in claims 1 or 2, **characterized in that** R₁ is selected from the group consisting of a glucosyl group, a galactosyl group and a rhamnosyl group.

4. The compound of formula (I) as claimed in any one of claims 1 to 3, **characterized in that** X is an oxygen atom.

5. The compound of formula (I) as claimed in any one of claims 1 to 3, **characterized in that** X is a sulfur atom.

6. A compound of formula (II):

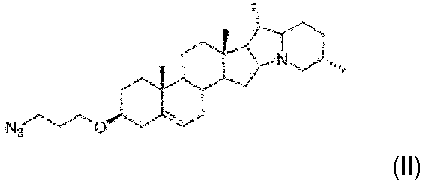

7. The non-therapeutic use of the compound of formula (I) as claimed in any one of claims 1 to 5 as an insecticide.

8. A method for obtaining the compound of formula (I) as claimed in any one of claims 1 to 5, wherein:
a) there is provided a compound of formula (II): 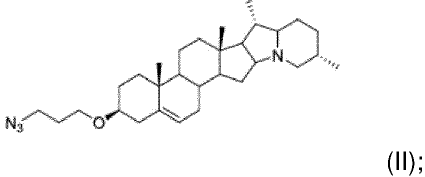 and
b) the compound of formula (II) is reacted, in the presence or absence of a catalyst, with a compound of formula (III):

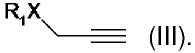

9. The method for obtaining the compound of formula (I) as claimed in claim 8, wherein:
c) there is provided said compound of formula (IV): 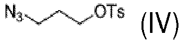 wherein Ts is a tosyl group;
d) there is provided a compound of formula (V): 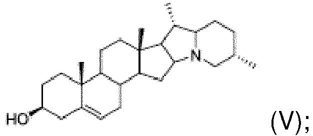 and
e) the compound of formula (V) is reacted with the compound of formula (IV) in order to obtain the compound of formula (II).

10. The method for obtaining the compound of formula (I) as claimed in claims 8 or 9, **characterized in that** said catalyst is selected from ruthenium compounds or copper compounds.

11. A composition comprising the compound of formula (I) as claimed in any one of claims 1 to 5.
